# FASCICULE DE BREVET EUROPEEN

(11) **EP 0 679 631 B1**
(45) Date de publication et mention de la délivrance du brevet: **06.05.1998**
(21) Numéro de dépôt: 95400737.3
(22) Date de dépôt: 03.04.1995
(51) Int. Cl.: C07C 65/28, A61K 31/19, C07C 65/19

(54) **Composés bi-aromatiques acétylénés à groupement adamantyle, compositions pharmaceutiques et cosmétiques les contenant et utilisations**
Biaromatische Verbindungen mit acetylenischer Dreifachbindung und Adamantylgruppe, sie enthaltende pharmazeutische und kosmetische Zusammensetzungen und Verwendungen
Biaromatic acetylated, an adamantyl group containing compounds, pharmaceutical and cosmetic compositions containing them and their use

(30) Priorité: 26.04.1994 FR 9405018
(43) Date de publication de la demande: 02.11.1995
(73) Titulaire: CENTRE INTERNATIONAL DE RECHERCHES DERMATOLOGIQUES GALDERMA, ( CIRD GALDERMA), 06560 Valbonne (FR)
(72) Inventeur: Bernardon, Jean-Michel, F-06650 Le Rouret (FR); Charpentier, Bruno, F-06410 Biot (FR)
(74) Mandataire: Tezier Herman, Béatrice

(56) Documents cités:
- EP-A- 0 176 034
- CHEMICAL ABSTRACTS, vol. 87, no. 3, 18 Juillet 1977, Columbus, Ohio, US; abstract no. 22693b, MUSANTAEVA,SH. ET AL. 'SYNTHESIS OF P-ACETYLENYLBENZOIC ACID' page 599 ;colonne 2 ; & DEPOSITED DOC., 1974 pages 297 - 75 VINITI

## Description

La présente invention concerne, à titre de produits industriels nouveaux et utiles, des composés bi-aromatiques présentant à la fois une insaturation de type acétylénique et un groupement de type adamantyle. Elle concerne également l'utilisation de ces nouveaux composés dans des compositions pharmaceutiques destinées à un usage en médecine humaine ou vétérinaire, ou bien encore dans des compositions cosmétiques.

Dans la demande de brevet EP 0176034 et dans le Chemical Abstract, vol. 87, no. 3; abst. no. 87:22693b, il a été décrit des composés mono ou bi-aromatiques présentant une insaturation acétylénique, mais ne présentant pas un groupement adamantyle.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies. Les composés selon l'invention peuvent par ailleurs trouver une application intéressante dans le traitement de l'ostéoporose, ou dans le traitement des maladies virales, ainsi que dans le traitement de tout état associé à une hypervitaminose A. D'une manière générale, ils peuvent enfin trouver une application dans le traitement de toute maladie qui est associée à une modification de l'expression des récepteurs appartenant à la superfamille des récepteurs des hormones stéroïdiennes et thyroïdiennes.

On peut également utiliser les composés selon l'invention dans des compositions cosmétiques pour l'hygiène corporelle et capillaire.

Les composés selon l'invention peuvent être représentés par la formule générale (I) suivante : 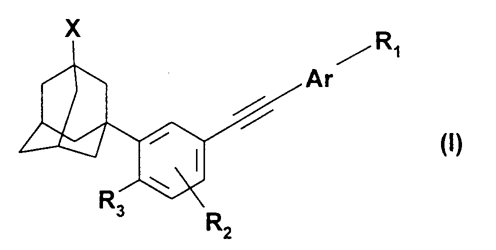 dans laquelle :
∗ X représente un atome d'hydrogène ou un atome d'halogène,
∗ R₁ représente un atome d'hydrogène, un radical -CH₃, un radical -CH₂-O-R₄, un radical -CH₂-O-CO-R₅, un radical -O-R₆, un radical -O-(CH₂)ₘ-(CO)ₙ-R₇, un radical -(CH₂)ₚ-CO-R₈, un radical -(CH₂)ₚ-CO-O-R₉, ou encore un radical -S(O)ₚ-R₁₀, les valeurs m, n et p ainsi que les différents radicaux R₄ à R₁₀ ayant la signification donnée ci-après,
* Ar représente un radical choisi parmi les radicaux de formules (a)-(f) suivantes : 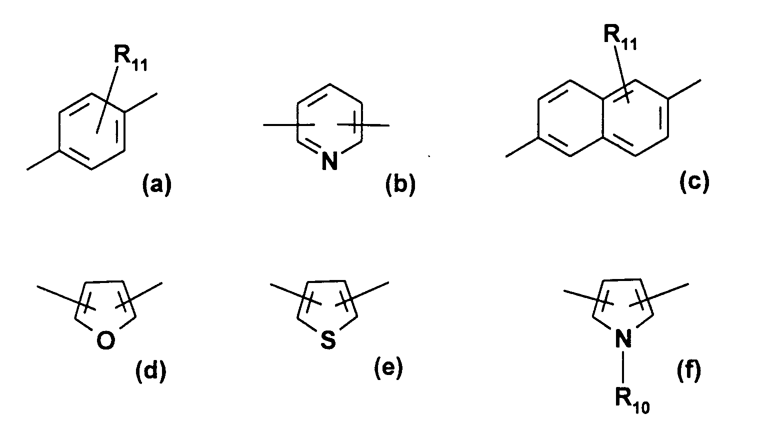 R₁₀ et R₁₁ ayant la signification donnée ci-après,
∗ R₂ représente un atome d'hydrogène ou un atome d'halogène, un radical alkyle, linéaire ou ramifié, ayant de 1 à 20 atomes de carbone, un radical -O-CH₂-O-CH₂-CH₂-O-CH₃ ou encore un radical -O-R₁₃, R₁₃ ayant la signification donnée ci-après,
∗ R₃ représente un radical -O-CH₂-O-CH₂-CH₂-O-CH₃, un radical -(Y)ₙ-(CH₂)_{q}-R₁₄, un radical -(CH₂)ₘ-Y-(CH₂)_{q}-R₁₄ ou bien encore -CH=CH-(CH₂)ₜ-R₁₄, les valeurs m, n, q et t et les radicaux Y et R₁₄ ayant la signification donnée ci-après,
   étant entendu que dans tout ce qui précède :
   - m, identique ou différent, est un nombre entier égal à 1, 2 ou 3, n, identique ou différent, est un nombre entier égal à 0 ou 1, p est un nombre entier égal à 0, 1, 2 ou 3, q est un nombre entier compris inclusivement entre 0 et 12, et t est un nombre entier compris inclusivement entre 0 et 10,
   - R₄ représente un atome d'hydrogène ou un radical alkyle inférieur,
   - R₅ représente un radical alkyle inférieur,
   - R₆ représente un atome d'hydrogène ou un radical alkyle inférieur,
   - R₇ représente un radical alkyle inférieur ou un hétérocycle,
   - R₈ représente un atome d'hydrogène, un radical alkyle inférieur ou un radical : 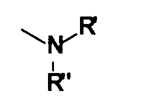 R' et R'', identiques ou différents, représentent un atome d'hydrogène, un radical alkyle inférieur, un radical mono ou polyhydroxyalkyle, un radical aryle éventuellement substitué ou un reste d'aminoacide ou de peptide ou de sucre ou encore, pris ensemble, forment un hétérocycle,
   - R₉ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
   - R₁₀ représente un atome d'hydrogène ou un radical alkyle inférieur,
   - R₁₁ représente un atome d'hydrogène, un atome d'halogène, un radical alkyle inférieur, un radical hydroxy, un radical -O-R₁₂ ou -O-COR₁₂, R₁₂ ayant la signification donnée ci-dessous,
   - R₁₂ représente un radical alkyle inférieur,
   - R₁₃ représente un atome d'hydrogène ou un radical alkyle, linéaire ou ramifié, ayant de 1 à 20 atomes de carbone,
   - R₁₄ représente un atome d'hydrogène, un radical alkyle inférieur, un radical alkényle, un radical alkynyle, un radical cycloaliphatique en C₃-C₆, un radical mono ou polyhydroxyalkyle dont les hydroxyles sont éventuellement protégés sous forme de méthoxy, d'acétoxy ou d'acétonide, un radical aryle ou aralkyle, un radical -CO-R₈, un radical -COOR₉, un radical -S(O)ₚ-R₁₀, un radical : 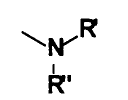 ou, mais seulement lorsque n est égal à 0 avec la signification de R₃ étant -(Y)ₙ-(CH₂)_{q}-R₁₄, un radical hydroxy, un radical -O-R₁₂, un radical -O-COR₁₂,
   - Y représente un atome d'oxygène, de soufre ou un radical -S(O)ₚ.

L'invention vise également les sels des composés de formule (I) ci-dessus dans les cas où le radical R₁ ou le radical R₁₄ représente une fonction acide carboxylique ou une fonction acide sulfonique ou encore lorsque ledit radical R₁₄ représente une fonction amine, ainsi que les analogues chiraux desdits composés. Lorsque les composés selon l'invention se présentent sous forme de sels, il s'agit de préférence de sels d'un métal alcalin ou alcalino-terreux, ou encore de zinc ou d'une amine organique.

Selon la présente invention, on entend par radical alkyle inférieur un radical ayant de 1 à 6 atomes de carbone, de préférence les radicaux méthyle, éthyle, isopropyle, butyle, tertiobutyle et hexyle.

Par radical alkyle linéaire ou ramifié ayant de 1 à 20 atomes de carbone, on entend notamment les radicaux méthyle, éthyle, propyle, 2-éthyl-hexyle, octyle, dodécyle, hexadécyle et octadécyl.

Par radical monohydroxyalkyle, on entend un radical ayant de préférence 2 ou 3 atomes de carbone, notamment un radical 2-hydroxyéthyle, 2-hydroxypropyle ou 3-hydroxypropyle.

Par radical polyhydroxyalkyle, on entend un radical contenant de préférence de 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle, on entend de préférence un radical phényle éventuellement substitué par au moins un atome d'halogène, un hydroxyle ou une fonction nitro.

Par radical aralkyle, on entend de préférence le radical benzyle ou phénéthyle éventuellement substitué par au moins un atome d'halogène, un hydroxyle ou une fonction nitro.

Par radical alkényle, on entend un radical contenant de préférence de 2 à 5 atomes de carbone et présentant une ou plusieurs insaturations éthyléniques, tel que plus particulièrement le radical allyle.

Par radical alkynyle, on entend un radical contenant de 2 à 6 atomes de carbone, tel que le radical propargyle.

Par reste de sucre, on entend un reste dérivant notamment de glucose, de galactose ou de mannose, ou bien encore de l'acide glucuronique.

Par reste d'aminoacide, on entend notamment un reste dérivant de la lysine, de la glycine ou de l'acide aspartique, et par reste de peptide on entend plus particulièrement un reste de dipeptide ou de tripeptide résultant de la combinaison d'acides aminés.

Par hétérocycle enfin, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Lorsque les radicaux X, R₂ et R₁₁ représentent un atome d'halogène, celui-ci est de préférence un atome de fluor, de chlore ou de brome.

Parmi les composés de formule (I) ci-dessus rentrant dans le cadre de la présente invention, on peut notamment citer les suivants :
Acide 4-[3-(1-adamantyl)-4-methoxyphenylethynyl]benzoïque
Acide 4-[3-(1-adamantyl)-4 methoxyethoxymethoxyphenylethynyl]benzoïque
Acide 4-[3-(1-adamantyl)-4-hydroxyphenylethynyl]benzoïque
Acide 5-[3-(1-adamantyl)-4-methoxyphenylethynyl]-2-thiophenecarboxylique
Acide 5-[3-(1-adamantyl)-4-hydroxyphenylethynyl]-2-thiophenecarboxylique
Acide 2-[3-(1-adamantyl)-4-methoxyphenylethynyl]-4-thiophenecarboxylique
Acide 6-[3-(1-adamantyl)-4-methoxyphenylethynyl]-2-naphtoïque
Acide 4-[3-(1-adamantyl)-4-nonyloxyphényléthynyl]benzoïque
Acide 4-[3-(1-adamantyl)-4-héxyloxyphényléthynyl]benzoïque
Acide 4-[3-(1-adamantyl)-4-dodécyloxyphényléthynyl]benzoïque
Acide 4-[3-(1-adamantyl)-4-cyclopropylméthoxyphényléthynyl]benzoïque
Acide 4-[2-héxyloxy-5-(1-adamantyl)-4-héxyloxyphényléthynyl]benzoïque
Acide 4-[3-(1-adamantyl)-4-heptyloxyphényléthynyl]benzoïque
Acide 6-[3-(1-adamantyl)-4-methoxyethoxymethoxyphenylethynyl]nicotinique
Acide 4-[2-methoxy-5-(1-adamantyl)-4-methoxyphenylethynyl]benzoïque
Acide 4-[2-méthoxyéthoxyméthoxy-5-(1-adamantyl)-4-méthoxyéthoxy méthoxyphenyléthynyl]benzoïque
Acide 4-[2-methoxy-5-(1-adamantyl)-4-methoxyethoxymethoxyphenyl ethynyl]benzoïque
Acide 4-[3-(1-adamantyl)-4-benzyloxyphenylethynyl]benzoïque
4-[3-(1-adamantyl)-4-méthoxyéthoxyméthoxyphényléthynyl]benzaldéhyde
4-[3-(1-adamantyl)-4-méthoxyéthoxyméthoxyphényléthynyl]benzèneméthanol
Acétate de 4-[3-(1-adamantyl)-4-méthoxyéthoxyméthoxyphényléthynyl]benzyle
Acétate de 4-[3-(1-adamantyl)-4-methoxyethoxymethoxyphenylethynyl]phényle
4-[3-(1-adamantyl)-4-méthoxyéthoxyméthoxyphényléthynyl]phénol
4-[3-(1-adamantyl)-4-méthoxyéthoxyméthoxyphényléthynyl]phénoxy éthylmorpholine
4-[3-(1-adamantyl)-4-méthoxyéthoxyméthoxyphényléthynyl]benzamide
N-éthyl-4-[3-(1-adamantyl)-4-méthoxyéthoxyméthoxyphényléthynyl]benzamide
Morpholide de l'acide 4-[3-(1-adamantyl)-4-methoxyethoxymethoxyphenyl ethynyl]benzoïque
Acide 2-hydroxy-4-[3-(1-adamantyl)-4-methoxyethoxymethoxyphenyl ethynyl]benzoïque
Acide 4-[3-(1-adamantyl)-4-(6-hydroxyhéxyloxy)phényléthynyl]benzoïque
Acide 4-[3-(1-adamantyl)-4-(6-méthoxyhéxyloxy)phényléthynyl]benzoïque
Acide 4-[[3-(1-adamantyl)-4-[2-(4-morpholino)éthoxy]phényléthynyl]]benzoïque
Acide 4-[3-(1-adamantyl)-4-(3-carbamoylpropyloxy)phényléthynyl]benzoïque
Acide 4-[3-(1-adamantyl)-4-(5-carbamoylpentyloxy)phényléthynyl]benzoïque
Acide 4-[3-(1-adamantyl)-4-(3-hydroxypropyloxy)phényléthynyl]benzoïque
Acide 4-[3-(1-adamantyl)-4-(3-hydroxy-2-méthylpropyloxy)phényléthynyl]benzoïque
Acide 4-[3-(1-adamantyl)-4-(2,2-diméthyl-1,3-dioxolanne-4-méthoxy)phényl éthynyl]benzoïque
Acide 4-[3-(1-adamantyl)-4-(2,3-dihydroxypropyloxy)phényléthynyl]benzoïque
Acide 4-[3-(1-adamantyl)-4-méthoxyéthoxyéthylphényléthynyl]benzoïque
Acide 4-[3-(1-adamantyl)-4-méthoxyméthoxypropylphényléthynyl] benzoïque
Acide 4-[3-(1-adamantyl)-4-méthoxyéthoxypropylphényléthynyl]benzoïque
Acide 4-[3-(1-adamantyl)-4-acétoxybutoxyphényléthynyl]benzoïque
Acide 4-[3-(1-adamantyl)-4-acétoxypropyloxyphényléthynyl]benzoïque.

Selon la présente invention, les composés de formule (I) plus particulièrement préférés sont ceux pour lesquels l'une au moins, et de préférence toutes, les conditions ci-dessous sont respectées :
- R₁ est un radical -(CH₂)ₚ-CO-R₈
- R₃ est un radical -O-CH₂-O-CH₂-CH₂-O-CH₃ ou -(Y)ₙ-(CH₂)_{q}-R₁₄
- Ar est un radical choisi parmi les radicaux de formule (a) et (e)
- et R₂ est un radical alkyle, linéaire ou ramifié, ayant de 1 à 20 atomes de carbone ou encore un radical -O-R₁₃.

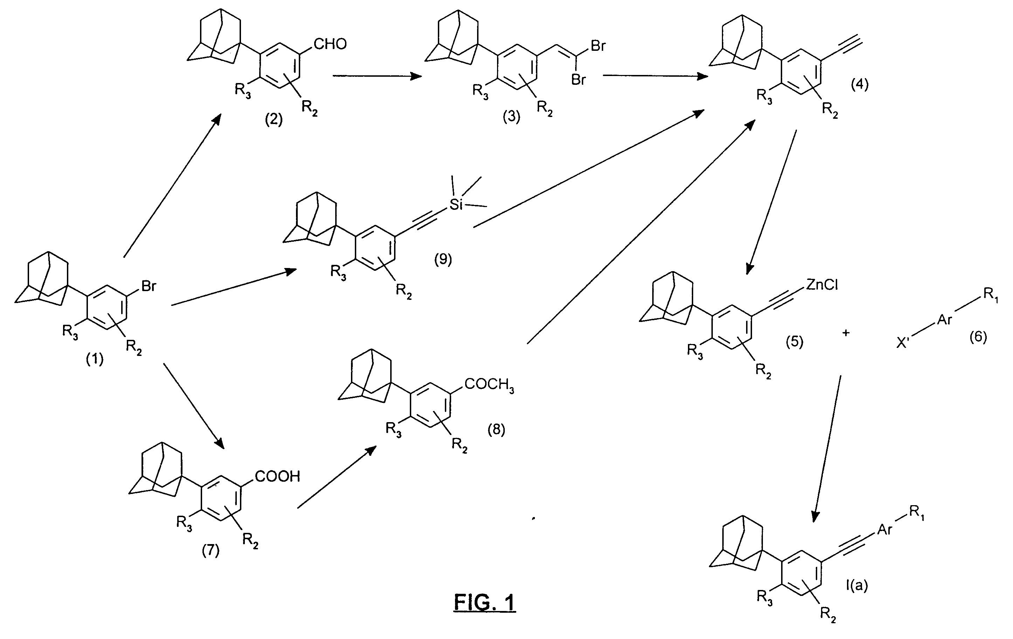

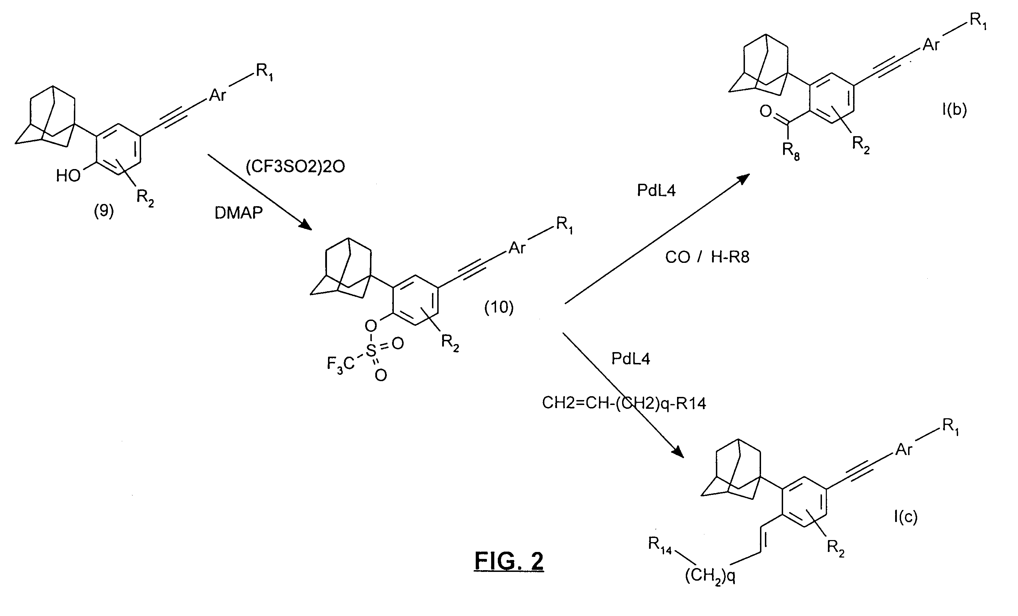

La présente invention a également pour objet les procédés de préparation des composés de formule (I), en particulier selon les schémas réactionnels donnés aux figures 1 et 2.

Ainsi, les composés de formule I(a) peuvent être obtenus (figure 1) à partir du dérivé acetylénique (4), par transformation en dérivé lithien puis zincique (5). Ce dernier est ensuite couplé avec le dérivé halogéné (6) (de préférence le dérivé iodé ou bromé) en présence d'un catalyseur au palladium (de préférence le tétrakis(triphenylphosphine)palladium(0)), dans un solvant tel que le THF.

Le dérivé acétylénique (4) peut, quant à lui, être obtenu à partir du dérivé bromé (1) par trois méthodes différentes :
- soit par transformation en composé aldéhydique (2) par réaction du dérivé magnésien ou lithien avec la dimethylformamide. L'aldéhyde (2) réagit ensuite avec le tetrabromure de carbone et la triphenylphosphine pour donner un dérivé 2',2'-dibromostyrene (3) qui est ensuite transformé en dérivé acetylenique (4) par une base non nucléophile telle que le n-butyllithium dans un solvant aprotique tel que le tetrahydrofuranne ;
- soit par transformation en dérivé acide (7) par réaction du dérivé magnésien ou lithien avec du CO₂. L'acide (7), en présence d'un large excès de methyllithium, est ensuite transformé en un dérivé cétonique (8) qui, par une suite de réactions (traitement avec une base telle que le diisopropylamidure de lithium puis avec un chlorure de dialkylphosphate et de nouveau avec le diisopropylamidure de lithium) est enfin transformé en dérivé acétylénique (4) ;
- soit par transformation en dérivé trimethylsilylacetylenique (9) par réaction avec le triméthylsilylacetylene dans un solvant tel que la triethylamine en présence d'acetate de palladium et de triphenylphosphine, puis désilylation en présence soit de carbonate de potassium soit de fluorure de tetrabutylammonium.

Dans les formules et réactions ci-dessus, R₁, R₂, R₃ ont les mêmes significations que celles données ci-avant pour la formule générale (I) ou en sont des dérivés convenablement protégés pour être compatibles avec les conditions de couplage. En particulier lorsqu'ils représentent le radical hydroxy, celui-ci est protégé de préférence sous forme de tert-butyldimethylsilyloxy ou de methoxyethoxymethoxy. La déprotection est alors effectuée soit en présence de fluorure de tetrabutylammonium ou d'iodure de trimethylsilane soit en milieu acide (par exemple HCl).

Lorsque R₃ est un radical -(CH₂)_{q}- CO-R₈ ou un radical -CH=CH-(CH₂)ₜ-R₁₄, les composés sont préparés préférentiellement à partir du dérivé phenolique (9) selon le schéma réactionnel donné à la figure 2, c'est à dire conversion du dérivé phenolique (9) en triflate (10) puis substitution nucléophile en présence d'un catalyseur au palladium (par exemple le tetrakis(triphenylphosphine)palladium(0)) selon les conditions générales décrites par S. Cacchi et al. dans Tetrahedron Letter 1986, 27 3931-4, ou par W. J. Scott et al. dans J. Org. Chem. 1985 50 2302-8.

La présente invention a également pour objet à titre de médicament les composés de formule (I) telle que définie ci-dessus.

Selon la nature des radicaux retenus, ces composés pourront présenter soit une activité agoniste dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer Research 38, p. 793-801, 1978), soit, au contraire, une activité antagoniste vis à vis de l'expression d'un ou plusieurs marqueurs biologiques dans le test de différenciation des cellules (F9) de tératocarcinome embryonnaire de la souris (Skin Pharmacol., 3, pp 256-267, 1990) et/ou sur la différenciation des kératinocytes humains in vitro (Anal. Biochem., 192, pp 232-236, 1991).

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acnée ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose,
17) dans le traitement ou la prévention de l'ostéoporose.

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutase, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses analogues chiraux ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses analogues chiraux ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses analogues chiraux ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,5-diphényl-imidazolidine 2,4-dione); des agents anti-inflammatoires non stéroïdiens; des caroténoïdes et, notamment, le β-caroténe; des agents anti-psoriatiques tels que l'anthraline et ses dérivés; et enfin les acides eicosa-5,8,11,14-tétraynoïque et eicosa-5,8,11-triynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés.

### EXEMPLE 1

### Acide 4-[3-(1-adamantyl)-4-methoxyphenylethynyl]benzoïque

### (a) 3-(1-adamantyl)-4-methoxyphenylacetylene

Dans un tricol et sous courant d'azote, on introduit 4,7 ml (33 mmoles) de diisopropylamine et 40 ml de THF. A -78°C, on ajoute goutte à goutte 20,6 ml (33 mmoles) de n-butyllithium (1,6 M dans de l'hexane) et agite à cette même température pendant 15 minutes. On ajoute ensuite une solution de 8,5 g (30 mmoles) de 3-(1-adamantyl)-4-methoxyacetophenone dans 50 ml de THF. Après agitation pendant une heure à -78°C, la solution est traitée avec 4,8 ml (33 mmoles) de diethylchlorophosphate et on laisse remonter à température ambiante. Cette solution est transférée à une solution de diisopropylamidure de lithium qui a été préparée en utilisant 9,35 ml (66 mmoles) de diisopropylamine et 41 ml (66 mmoles) de n-butyllithium (1,6 M dans l'hexane) dans 90 ml de THF à - 78°C. On laisse remonter à température ambiante et agite douze heures. On verse le milieu réactionnel dans de l'eau glacée et on ajuste le pH à 1 avec de l'acide chlorhydrique (5N). On extrait avec de l'éther éthylique, décante la phase organique, sèche sur sulfate de magnésium, évapore. Le résidu obtenu est purifié par chromatographie sur colonne de silice élué avec un mélange de dichlorométhane et d'hexane (10-90). Après évaporation des solvants, on recueille 4,3 g (54%) du dérivé acetylenique attendu de point de fusion 121-2°C.

### (b) Methyl 4-[3-(1-adamantyl)-4-methoxyphenylethynyl]benzoate

A une solution de 1,8 g (6,8 mmoles) de 3-(1-adamantyl)-4-methoxyphenylacetylene dans 20 ml de THF à 0°C, on ajoute goutte à goutte 4,4 ml (7 mmoles) de n-butyllithium (1,6 M dans l'hexane), et on agite trente minutes à température ambiante. On refroidit à 0°C et on ajoute 1,05 g (7,7 mmoles) de ZnCl₂ anhydre et agite pendant une heure à température ambiante.

Dans un tricol, on introduit 1,3 g (1,1 mmole) de tetrakis(triphenylphosphine)Palladium(0), 13 ml de THF, et on ajoute une solution de 1,77 g (6,7 mmoles) de methyl 4-iodobenzoate. On agite à température ambiante pendant trente minutes et on ajoute goutte à goutte la solution de zincique préparée précédemment. Le mélange réactionnel est agité à température ambiante pendant douze heures, puis versé dans un mélange de 80 ml d'acide chlorhydrique (3N) et d'eau glacée et enfin extrait avec de l'acétate d'ethyle. On décante la phase organique, lave à l'eau, sèche sur sulfate de magnésium, évapore. Le résidu obtenu est purifié par chromatographie sur colonne de silice élué avec un mélange d'hexane et d'éther éthylique (90-10). Après évaporation des solvants, on recueille 970 mg (36%) d'ester methylique attendu de point de fusion 197-8°C.

### (c) Acide 4-[3-(1-adamantyl)-4-methoxyphenylethynyl]benzoïque

Dans un ballon, on introduit 966 mg (2,4 mmoles) de l'ester préparé au point (b) ci-dessus, 20 ml de soude méthanolique (2N) et on chauffe à reflux pendant une heure. On évapore à sec le milieu réactionnel, reprend par l'eau, acidifie avec de l'acide chlorhydrique concentré, filtre le solide qui a précipité. Après recristallisation dans l'alcool éthylique, on recueille 660 mg (71%) de l'acide attendu de point de fusion 307-10°C.

### EXEMPLE 2

### Acide 4-[3-(1-adamantyl)-4-methoxyethoxymethoxyphenylethynyl]benzoïque

### (a) 3-(1-adamantyl)-1-bromo-4-methoxyethoxymethoxybenzene

Dans un tricol et sous courant d'azote on introduit 3,8 g (0,13 mole) d'hydrure de sodium (80% dans l'huile) et 50 ml de DMF, puis on ajoute goutte à goutte une solution de 40 g (0,13 mole) de 2-(1-adamantyl)-4-bromophenol dans 100 ml de DMF, et on agite jusqu'a cessation du dégagement gazeux. On ajoute ensuite goutte à goutte une solution de 18 ml (0,15 mole) de chlorure de 2-methoxyethoxymethyl dans 20 ml de DMF et on agite pendant quatre heures à température ambiante. On verse ensuite le milieu réactionnel dans l'eau, extrait avec de l'éther éthylique, décante la phase organique, lave à l'eau, sèche sur sulfate de magnésium, et évapore. Le résidu est purifié par chromatographie sur colonne de silice élué avec un mélange de dichlorométhane et d'hexane (50-50). Après évaporation des solvants, on recueille 40,1 g (78%) du produit attendu de point de fusion 69-70°C.

### (b) Acide 3-(1-adamantyl)-4-methoxyethoxymethoxybenzoïque

Le composé obtenu au point (a) ci-dessus (28,5 g, 72 mmoles) est dissout dans 200 ml de THF. La solution obtenue est ajoutée goutte à goutte sur du magnésium (2,4 g, 100 mmoles) et un cristal d'iode. Après introduction, on chauffe à reflux pendant deux heures, refroidit à -78°C et fait passer un courant de CO₂ pendant une heure. On laisse remonter à température ambiante, verse le milieu réactionnel dans une solution aqueuse saturée en chlorure d'ammonium, extrait avec de l'éther éthylique, décante la phase organique, sèche sur sulfate de magnésium, évapore. Le résidu obtenu est trituré dans l'hexane, filtré et sèché. On recueille 15,5 g (60%) de l'acide attendu de point de fusion 115-6°C.

### (c) 3-(1-adamantyl)-4-methoxyethoxymethoxyacetophenone

Dans un tricol et sous courant d'azote, on introduit 15,5 g (43 mmoles) d'acide 3-(1-adamantyl)-4-methoxyethoxymethoxybenzoïque et 300 ml d'éther éthylique anhydre. A -20°C, on ajoute goutte à goutte 80 ml (0,13 mole) de methyllithium (1,6 M dans l'ether) puis agite pendant trois heures à température ambiante. On verse le milieu réactionnel dans une solution aqueuse saturée en chlorure d'ammonium, décante la phase organique, sèche sur sulfate de magnésium, évapore. On recueille 15,4 g (100%) de l'acetophenone attendu sous forme d'une huile légèrement jaune.

### (d) 3-(1-adamantyl)-4-methoxyethoxymethoxyphenylacetylene

De manière analogue à l'exemple 1(a) ci-dessus, à partir de 11,8 g (33 mmoles) de 3-(1-adamantyl)-4-methoxyethoxymethoxyacetophenone, on obtient 6,3 g (57%) de l'acetylenique attendu de point de fusion 81-2°C.
(d) Methyl 4-[3-(1-adamantyl)-4-methoxyethoxymethoxyphenylethynyl]benzoate

De manière analogue à l'exemple 1(b) ci-dessus, par réaction de 1,5 g (4,4 mmoles) de 3-(1-adamantyl)-4-methoxyethoxymethoxyphenylacetylene avec 1,2 g (4,4 mmoles) de methyl 4-iodobenzoate et après purification par chromatographie sur colonne de silice élué avec un mélange de dichlorométhane et d'hexane (70-30), on obtient 1,5 g (72%) de l'ester methylique attendu de point de fusion 92-3°C.

### (e) Acide 4-[3-(1-adamantyl)-4-methoxyethoxymethoxyphenylethynyl]benzoïque

De manière analogue à l'exemple 1(c) ci-dessus, à partir de 730 mg (1,5 mmoles) de l'ester préparé au point (d) précédent, on obtient 600 mg (87%) de l'acide attendu de point de fusion 203-4°C.

### EXEMPLE 3

### Acide 4-[3-(1-adamantyl)-4-hydroxyphenylethynyl]benzoïque

### (a) Methyl 4-[3-(1-adamantyl)-4-hydroxyphenylethynyl]benzoate

Dans un ballon, on introduit 1,3 g (2,7 mmoles) de methyl 4-[3-(1-adamantyl)-4-methoxyethoxymethoxyphenylethynyl]benzoate et 50 ml de dichloromethane anhydre, et à 0°C on ajoute goutte à goutte 2,7 ml (2,7 mmoles) de trichlorure de bore (1 M dans CH₂Cl₂). On agite à température ambiante pendant une heure, verse le milieu réactionnel dans l'eau, extrait avec de l'éther éthylique, décante la phase organique, sèche sur sulfate de magnésium, et évapore. Le résidu obtenu est trituré dans un mélange d'hexane et d'éther éthylique, filtré et sèché. On recueille 910 mg (91%) du produit attendu de point de fusion 232-3°C.

### (b) Acide 4-[3-(1-adamantyl)-4-hydroxyphenylethynyl]benzoïque

De manière analogue à l'exemple 1(c) ci-dessus, à partir de 850 mg (2,2 mmoles) de methyl 4-[3-(1-adamantyl)-4-hydroxyphenylethynyl]benzoate, on recueille 720 mg (88%) de l'acide attendu de point de fusion 254-5°C.

### EXEMPLE 4

### Acide 5-[3-(1-adamantyl)-4-hydroxyphenylethynyl]-2-thiophenecarboxylique

### (a) Methyl 5-[3-(1-adamantyl)-4-methoxyethoxymethoxyphenymlethynyl]-2-thiophenecarboxylate

De manière analogue à l'exemple 1(b) ci-dessus, par réaction de 1,5 g (4,4 mmoles) de 3-(1-adamantyl)-4-methoxyethoxymethoxyphenylacetylene avec 1 g (4,4 mmoles) de methyl 5-bromo-2-thiophenecarboxylate, on obtient 1,4 g (67%) de l'ester methylique attendu sous forme d'une huile.

### (b) 5-[3-(1-adamantyl)-4-hydroxyphenylethynyl]-2-thiophenecarboxylate de méthyle

De manière analogue à l'exemple 3(a) ci-dessus, à partir de 1,2 g (2,5 mmoles) de l'ester obtenu au point (a) précédent, on obtient 847 mg (86%) de 5-[3-(1-adamantyl)-4-hydroxyphenylethynyl]-2-thiophenecarboxylate de méthyle de point de fusion 207-8°C.

### (c) Acide 5-[3-(1-adamantyl)-4-hydroxyphenylethynyl]-2-thiophenecarboxylique

De manière analogue à l'exemple 1(c) ci-dessus, à partir de 360 mg (0,9 mmole) de l'ester methylique obtenu au point (b) précédent, on obtient 300 mg (86%) d'acide 5-[3-(1-adamantyl)-4-hydroxyphenylethynyl]-2-thiophene carboxylique de point de fusion 249-50°C.

### EXEMPLE 5

### Acide 5-[3-(1-adamantyl)-4-methoxyphenylethynyl]-2-thiophenecarboxylique

### (a) 5-[3-(1-adamantyl)-4-methoxyphenylethynyl]-2-thiophenecarboxylate de méthyle

Dans un tricol et sous courant d'azote, on introduit 36 mg (1,2 mmole) d'hydrure de sodium (80% dans l'huile) et 20 ml de DMF, et on ajoute goutte à goutte une solution de 392 mg (1 mmole) de 5-[3-(1-adamantyl)-4-hydroxyphenylethynyl]-2-thiophenecarboxylate de méthyle dans 10 ml de DMF. On agite pendant trente minutes et ajoute ensuite 75 µl (1,2 mmole) d'iodomethane et agite à température ambiante pendant quatre heures. On verse ensuite le milieu réactionnel dans l'eau, extrait avec de l'éther éthylique, décante la phase organique, sèche sur sulfate de magnésium, évapore. Le résidu est trituré dans l'hexane, filtré, sèché. On recueille 385 mg (95%) du produit attendu de point de fusion 137-8°C.

### (b) Acide 5-[3-(1-adamantyl)-4-methoxyphenylethynyl]-2-thiophenecarboxylique

De manière analogue à l'exemple 1(c) ci-dessus, à partir de 370 mg (0,9 mmole) de l'ester methylique obtenu au point (a) précédent, on obtient 320 mg (90%) d'acide 5-[3-(1-adamantyl)-4-methoxyphenylethynyl]-2-thiophene carboxylique de point de fusion 244-5°C.

### EXEMPLE 6

### Acide 2-[3-(1-adamantyl)-4-methoxyphenylethynyl]-4-thiophenecarboxylique

### (a) 2-[3-(1-adamantyl)-4-methoxyphenylethynyl]-4-thiophenecarboxylate d'éthyle

De manière analogue à l'exemple 1(b) ci-dessus, par réaction de 3,2 g (12 mmoles) de 3-(1-adamantyl)-4-methoxyphenylacetylene avec 2,8 g (12 mmoles) 2-bromo-4-thiophenecarboxylate d'éthyle, on obtient, après chromatographie sur colonne de silice éluée avec un mélange d'hexane et d'acétate d'éthyle, 2,4 g (48%) de l'ester éthylique attendu de point de fusion 88-90°C.

### (b) Acide 2-[3-(1-adamantyl)-4-methoxyphenylethynyl]-4-thiophenecarboxylique

De manière analogue à l'exemple 1(c) ci-dessus, à partir de 2,3 g (5,4 mmoles) de l'ester obtenu au point (a) précédent, on obtient, après recristallisation dans l'alcool éthylique, 1,8 g (86%) d'acide 2-[3-(1-adamantyl)-4-methoxyphenylethynyl]-4-thiophenecarboxylique de point de fusion 244-6°C.

### EXEMPLE 7

### Acide 6-[3-(1-adamantyl)-4-methoxyphenylethynyl]-2-naphthoïque

### (a) 6-[3-(1-adamantyl)-4-methoxyphenylethynyl]-2-naphthoate de méthyle

De manière analogue à l'exemple 1(b) ci-dessus, à partir de 4,5 g (16,9 mmoles) de 3-(1-adamantyl)-4-methoxyphenylacetylene et de 4,45 g (16,8 mmoles) de 6-bromo-2-naphthoate de méthyle, on obtient 3,8 g (50%) de l'ester methylique attendu de point de fusion 233-4°C.

### (b) Acide 6-[3-(1-adamantyl)-4-methoxyphenylethynyl]-2-naphthoïque

De manière analogue à l'exemple 1(c) ci-dessus, à partir de 3,8 g (8,4 mmoles) de l'ester obtenu au point (a) précédent, on obtient 2,2 g (62%) d'acide 6-[3-(1-adamantyl)-4-methoxyphenylethynyl]-2-naphthoïque de point de fusion 307-9°C.

### EXEMPLE 8

### Acide 4-[3-(1-adamantyl)-4-nonyloxyphényléthynyl]benzoïque.

### (a) 4-[3-(1-adamantyl)-4-nonyloxyphényléthynyl]benzoate de méthyle.

Dans un tricol et sous courant d'azote, on introduit 75 mg (2,6 mmoles) d'hydrure de sodium (80% dans l'huile) et 10 ml de DMF. On ajoute goutte à goutte une solution de 1 g (2,6 mmoles) de 4-[3-(1-adamantyl)-4-hydroxyphényléthynyl] benzoate de méthyle dissous dans 70 ml de DMF et agite à température ambiante jusqu'à cessation du dégagement gazeux. On ajoute ensuite goutte à goutte 610 µl (3,1 mmoles) de 1-iodononane et agite à température ambiante pendant quatres heures. On verse le milieu réactionnel dans l'eau glacée, on extrait avec de l'éther éthylique, décante la phase organique, puis on sèche sur sulfate de magnésium, évapore. Le résidu obtenu est purifié par chromatographie sur colonne de silice élué avec un mélange de dichlorométhane et d'heptane (50-50). Après évaporation des solvants, on recueille 960 mg (72%) de l'ester méthylique attendu de point de fusion 112-5°C.

### (b) Acide 4-[3-(1-adamantyl)-4-nonyloxyphényléthynyl]benzoïque.

Dans un ballon, on introduit 960 mg (1,9 mmoles) de l'ester méthylique précédent et 25 ml d'une solution de soude méthanolique 2N et chauffe à reflux pendant deux heures. On évapore à sec le milieu réactionnel, reprend par l'eau acidifie à pH 1 avec de l'acide chlorhydrique, extrait avec de l'éther éthylique, décante la phase organique, sèche sur sulfate de magnésium, évapore. Le résidu obtenu est trituré dans un mélange de dichlorométhane et d'heptane (20-80), filtré et séché. On recueille 630 mg (67%) d'acide 4-[3-(1-adamantyl)-4-nonyloxyphényléthynyl] benzoïque de point de fusion 224-6°C.

### EXEMPLE 9

### Acide 4-[3-(1-adamantyl)-4-héxyloxyphényléthynyl]benzoïque.

### (a) 4-[3-(1-adamantyl)-4-héxyloxyphényléthynyl]benzoate de méthyle.

De manière analogue à l'exemple 8(a) par réaction de 1 g (2,6 mmoles) de 4-[3-(1-adamantyl)-4-hydroxyphényléthynyl]benzoate de méthyle avec 460 µl (3,1 mmoles) de 1-iodohexane, on obtient 900 mg d'ester méthylique attendu de point de fusion 140-3°C.

### (b) Acide 4-[3-(1-adamantyl)-4-héxyloxyphényléthynyl]benzoïque.

De manière analogue à l'exemple 8(b) à partir de 900 mg (1,9 mmoles) de l'ester méthylique précédent, on obtient 700 mg (80%) d'acide 4-[3-(1-adamantyl)-4-hexyloxyphényléthynyl]benzoïque de point de fusion 260-2°C.

### EXEMPLE 10

### Acide 4-[3-(1-adamantyl)-4-dodécyloxyphényléthynyl]benzoïque.

### (a) 4-[3-(1-adamantyl)-4-dodécyloxyphényléthynyl]benzoate de méthyle.

De manière analogue à l'exemple 8(a) par réaction de 1 g (2,6 mmoles) de 4-[3-(1-adamantyl)-4-hydroxyphényléthynyl]benzoate de méthyle avec 750 µl (3,1 mmoles) de 1-bromododécane, on obtient 1,1 g (77%) d'ester méthylique attendu de point de fusion 101-4°C.

### (b) Acide 4-[3-(1-adamantyl)-4-dodécyloxyphényléthynyl]benzoïque.

De manière analogue à l'exemple 8(b) à partir de 1,1 g (2 mmoles) de l'ester méthylique précédent, on obtient 1 g (93%) d'acide 4-[3-(1-adamantyl)-4-dodécyloxyphényléthynyl]benzoïque de point de fusion 225-7°C.

### EXEMPLE 11

### Acide 4-[3-(1-adamantyl)-4-cyclopropylméthoxyphényléthynyl]benzoïque.

### (a) 4-[3-(1-adamantyl)-4-cyclopropylméthoxyphényléthynyl]benzoate de méthyle.

De manière analogue à l'exemple 8(a) par réaction de 1 g (2,6 mmoles) de 4-[3-(1-adamantyl)-4-hydroxyphényléthynyl]benzoate de méthyle avec 300 µl (3,1 mmoles) de bromométhylcyclopropane, on obtient 700 mg (61 %) de l'ester méthylique attendu de point de fusion 185-6°C.

### (b) Acide 4-[3-(1-adamantyl)-4-cyclopropylméthoxyphényléthynyl]benzoïque.

De manière analogue à l'exemple 8(b) à partir de 700 mg (1,6 mmoles) de l'ester méthylique précédent, on obtient 570 mg (84%) d'acide 4-[3-(1-adamantyl)-4-cyclopropylméthoxyphenyléthynyl]benzoïque de point de fusion 302-5°C.

### EXEMPLE 12

### Acide 4-[2-hexyloxy-5-(1-adamantyl)-4-hexyloxyphenylethynyl]benzoïque.

### (a) 2,4-dihydroxy-5-(1-adamantyl)benzaldéhyde.

Dans un ballon, on introduit 5 g (36,2 mmoles) de 2,4-dihydroxybenzaldéhyde 5,8 g (38,1 mmoles) de 1-adamantanol et 200 ml de dichlorométhane. On ajoute 2 ml d'acide sulfurique concentré et agite à température ambiante pendant huit heures. On évapore à sec le milieu réactionnel, reprend par l'eau, neutralise avec du bicarbonate de sodium, extrait avec de l'éther éthylique, sèche sur sulfate de magnésium, évapore. On triture le résidu obtenu avec un mélange de dichlorométhane et d'heptane (40-60), filtre le solide et sèche. On recueille 6,8 g (69%) du produit attendu de point de fusion 165-8°C.

### (b) 2,4-dihexyloxy-5-(1-adamantyl)benzaldéhyde.

Dans un tricol et sous courant d'azote, on introduit 1,3 g (35,2 mmoles) d'hydrure de sodium (80% dans l'huile) et 50 ml de DMF. On ajoute goutte à goutte une solution de 4,8 g (17,6 mmoles) de 2,4-dihydroxy-5-(1-adamantyl)benzaldéhyde dissous dans 70 ml de DMF et agite à température ambiante jusqu'à cessation du dégagement gazeux. On ajoute ensuite goutte à goutte 6,5 ml (35,2 mmoles) de 1-iodohexane et agite à température ambiante quatres heures. On verse le milieu réactionnel dans eau glacée, extrait avec de l'éther éthylique, décante la phase organique, sèche sur sulfate de magnésium, évapore. Le résidu obtenu est purifié par chromatographie sur colonne de silice élué avec du dichlorométhane. Après évaporation des solvants, on recueille 5,5 g (71%) du produit attendu sous forme d'une huile légèrement bleue.

### (c) 2',2'-dibromo-2,4-dihexyloxy-5-(1-adamantyl)styrène.

Dans un ballon on introduit 5,5 g (12,5 mmoles) de 2,4-dihexyloxy-5-(1-adamantyl)benzaldéhyde et 100 ml de dichlorométhane. On ajoute successivement 8,3 g (25 mmoles) de tétrabromure de carbone 6,5 g (25 mmoles) de triphénylphosphine et 1,6 g (25 mmoles) de poudre de zinc et agite à température ambiante pendant deux heures. On évapore le milieu réactionnel et purifie le résidu obtenu par chromatographie sur colonne de silice élué avec du dichlorométhane. On recueille 3 g (40%) du produit attendu sous forme d'une huile jaune.

### (d) 2,4-dihexyloxy-5-(1-adamantyl)phénylacétylène

Dans un tricol et sous courant d'azote on introduit 3 g (5 mmoles) de 2',2'-dibromo-2,4-dihexyloxy-5-(1-adamantyl)styrène et 50 ml de THF. A -78°C on ajoute goutte à goutte 4 ml (10 mmoles) d'une solution de n-butyllithium (2,5 M dans l'hexane) et laisse remonter à température ambiante pendant une heure. On verse le milieu réactionnel dans l'eau, extrait avec de l'éther éthylique, décante la phase organique, sèche sur sulfate de magnésium, évapore. Le résidu obtenu est purifié par chromatographie sur colonne de silice élué avec du dichlorométhane. On recueille 1,9 g (86%) du dérivé acétylénique attendu sous forme d'une huile marron.

### (e) 4-[2-hexyloxy-5-(1-adamantyl)-4-hexyloxyphenylethynyl]benzoate de méthyle.

Dans un tricol, on introduit 1,8 g (4,1 mmoles) de 2,4-dihexyloxy-5-(1-adamantyl)phénylacétylène 1,2 g (4,5 mmoles) de 4-iodobenzoate de méthyle et 15 ml de triéthylamine. On dégaze le milieu réactionnel avec de l'azote pendant 15' et ajoute 100 mg d'iodure de cuivre et 260 mg (0,37 mmole) de chlorure de bis(triphénylphosphine)palladium(ll) et agite à température ambiante pendant douze heures. On évapore à sec le milieu réactionnel, reprend par l'eau et l'éther éthylique, décante la phase organique, sèche sur sulfate de magnésium, évapore. Le résidu obtenu est purifié par chromatographie sur colonne de silice élué avec un mélange de dichlorométhane et d'heptane (20-80). Aprés évaporation des solvants, on recueille 1,7 g (72%) de l'ester méthylique attendu.

### (f) acide 4-[2-hexyloxy-5-(1-adamantyl)-4-hexyloxyphenylethynyl]benzoïque.

De maniére analogue à l'exemple 8(b) à partir de 1,7 g (3 mmoles) de l'ester méthylique précédent, on obtient 1,5 g (90%) de l'acide attendu de point de fusion 213-4°C.

### EXEMPLE 13

### Acide 4-[3-(1-adamantyl)-4-heptyloxyphenylethynyl]benzoïque.

### (a) 4-[3-(1-adamantyl)-4-heptyloxyphenylethynyl]benzoate de méthyle

De manière analogue à l'exemple 8(a) par réaction de 500 mg (1,3 mmole) de 4-[3-(1-adamantyl)-4-hydroxyphenylethynyl]benzoate de méthyle avec 250 µl (1,56 mmole) de 1-bromoheptane,on obtient 500 mg (82%) d'ester méthylique attendu.

### (b) Acide 4-[3-(1-adamantyl)-4-heptyloxyphenylethynyl]benzoïque.

De manière analogue à l'exemple 8(b) à partir de 500 mg (1,07 mmole) de l'ester méthylique précédent, on obtient 350 mg (70%) d'acide 4-[3-(1-adamantyl)-4-heptyloxyphenylethynyl]benzoïque de point de fusion 256-7°C.

### EXEMPLE 14

### Acide 6-[3-(1-adamantyl)-4-méthoxyéthoxyméthoxyphényléthynyl]nicotinique.

### (a) 6-[3-(1-adamantyl)-4-méthoxyéthoxyméthoxyphényléthynyl]nicotinate de méthyle.

De manière analogue à l'exemple 12(e) par réaction de 2,5 g (7,4 mmoles) de 3-(1-adamantyl)-4-méthoxyéthoxyméthoxyphénylacétylène avec 1,93 g (7,4 mmoles) de 6-iodonicotinate de méthyle, on obtient 1,98 g (56%) de l'ester méthylique attendu de point de fusion 86-7°C.

### (b) acide 6-[3-(1-adamantyl)-4-methoxyethoxymethoxyphenylethynyl]nicotinique.

De manière analogue à l'exemple 8(b) à partir de 1,5 g (3,1 mmoles) de l'ester méthylique précédent, on obtient 1,4 g (95%) de l'acide attendu de point de fusion 204-5°C.

### EXEMPLE 15

### Acide 4-[2-methoxy-5-(1-adamantyl)-4-methoxyphenylethynyl]benzoïque.

### (a) 2,4-di-méthoxy-5-(1-adamantyl)benzaldéhyde.

De manière analogue à l'exemple 12(b) par réaction de 1,1 g (3,8 mmoles) de 2,4-dihydroxy-5-(1-adamantyl)benzaldéhyde avec 620 µl (9,8 mmoles) d'iodure de méthyle, on obtient 1 g (87%) du produit attendu de point de fusion 152-4°C.

### (b) 2',2'-di-bromo-2,4-diméthoxy-5-(1-adamantyl)styrène.

De manière analogue à l'exemple 12(c) à partir de 1,8 g (6 mmoles) de l'aldéhyde précédent, on obtient 1,2 g (44%) de 2',2'-dibromo-2,4-di-méthoxy-5-(1-adamantyl)styrène de point de fusion 176-8°C.

### (c) 2,4-di-méthoxy-5-(1 -adamantyl)phénylacétylène.

De manière analogue à l'exemple 12(d) à partir de 1,2 g (2,6 mmoles) du produit précédent, on obtient 300 mg (38%) du dérivé acétylénique attendu de point de fusion 210-3°C.

### (d) 4-[2-méthoxy-5-(1 -adamantyl)-4-méthoxyphényléthynyl]benzoate de méthyle.

De manière analogue à l'exemple 12(e) par réaction de 300 mg (1 mmole) de 2,4-diméthoxy-5-(1-adamantyl)phénylacétylène avec 265 mg (1 mmole) de 4-iodobenzoate de méthyle, on obtient 350 mg (80%) de l'ester méthylique attendu de point de fusion 186-8°C.

### (e) acide 4-[2-méthoxy-5-(1-adamantyl)-4-méthoxyphényléthynyl]benzoïque.

De manière analogue à l'exemple 8(b) à partir de 350 mg (0,8 mmole) de l'ester méthylique précédent, on obtient 290 mg (85%) d'acide 4-[2-méthoxy-5-(1-adamantyl)-4-méthoxyphényléthynyl]benzoïque de point de fusion 274-8°C.

### EXEMPLE 16

### Acide 4-[2-méthoxyéthoxyméthoxy-5-(1-adamantyl)-4-méthoxyéthoxyméthoxy phenyléthynyl]benzoïque.

### (a) 2,4-di-méthoxyéthoxyméthoxy-5-(1-adamantyl)benzaldéhyde.

De manière analogue à l'exemple 12(b) par réaction de 25 g (92 mmoles) de 2,4-di-hydroxy-5-(1-adamantyl)benzaldéhyde avec 26,2 ml (230 mmoles) de chlorure de méthoxyéthoxyméthyle, on obtient 31 g (75%) du produit attendu sous forme d'une huile jaune.

### (b) 2',2'-dibromo-2,4-di-méthoxyéthoxyméthoxy-5-(1-adamantyl)styrène.

De manière analogue à l'exemple 12(c) à partir de 4,5 g (10 mmoles) de l'aldéhyde précédent, on obtient 5,5 g (90%) de 2',2'-dibromo-2,4-di-méthoxy éthoxyméthoxy-5-(1-adamantyl)styrène sous forme d'une huile marron.

### (c) 2,4-di-méthoxyéthoxyméthoxy-5-(1-adamantyl)phénylacétylène.

De manière analogue à l'exemple 12(d) à partir de 5,5 g (9,1 mmoles) du produit précédent, on obtient 900 mg (23%) du dérivé acétylénique attendu sous forme d'une huile incolore.

### (d) 4-[2-méthoxyéthoxyméthoxy-5-(1-adamantyl)-4-méthoxyéthoxyméthoxy phényléthynyl]benzoate de méthyle.

De manière analogue à l'exemple 12(e) par réaction de 900 mg (2 mmoles) de 2,4-diméthoxyéthoxyméthoxy-5-(1-adamantyl)phénylacétylène avec 530 mg (2 mmoles) de 4-iodobenzoate de méthyle, on obtient 950 mg (81%) de l'ester méthylique attendu sous forme d'une huile marron.

### (e) acide 4-[2-méthoxyéthoxyméthoxy-5-(1-adamantyl)-4-méthoxyéthoxyméthoxy phényléthynyl]benzoïque.

De manière analogue à l'exemple 8(b) à partir de 950 mg (1,6 mmole) de l'ester méthylique précédent, on obtient 730 mg (79%) d'acide 4-[2-méthoxyéthoxy méthoxy-5-(1-adamantyl)-4-méthoxyéthoxyméthoxyphényléthynyl]benzoïque de point de fusion 131-5°C.

### EXEMPLE 17

### Acide 4-[2-methoxy-5-(1-adamantyl)-4-methoxyethoxymethoxyphenyl ethynyl]benzoïque.

### (a) 2-hydroxy-4-méthoxyéthoxyméthoxy-5-(1-adamantyl)benzaldéhyde.

Dans un tricol et sous courant d'azote, on introduit 5 g (11,2 mmoles) de 2,4-diméthoxyéthoxyméthoxy-5-(1-adamantyl)benzaldéhyde et 100 ml de dichlorométhane. A -78°C, on ajoute goutte à goutte une solution de 11,2 ml de trichlorure de bore (1M dans l'hexane) et agite à cette même température pendant 18 heures. On verse le milieu réactionnel dans l'eau glacée, décante la phase organique, sèche sur sulfate de magnésium, évapore. Le résidu obtenu est purifié par chromatographie sur colonne de silice élué avec du dichlorométhane. On recueille après évaporation des solvants 3,1 g (77%) de 2-hydroxy-4-méthoxy éthoxyméthoxy-5-(1-adamantyl)benzaldéhyde de point de fusion 102-4°C.

### (b) 2-méthoxy-4-méthoxyéthoxyméthoxy-5-(1-adamantyl)benzaldéhyde.

De manière analogue à l'exemple 12(b) par réaction de 2,9 g (8 mmoles) du dérivé aldéhydique précédent avec 550 µl (8,8 mmoles) d'iodure de méthyle, on obtient 2,1 g (70%) du produit attendu de point de fusion 48-50°C.

### (c) 2',2'-dibromo-2-méthoxy-4-méthoxyéthoxyméthoxy-5-(1-adamantyl)styrène.

De manière analogue à l'exemple 12(c) à partir de 2,1 g (5,6 mmoles) de l'aldéhyde précédent, on obtient 2 g (67%) de 2',2'-dibromo-2-méthoxy-4-méthoxyéthoxyméthoxy-5-(1-adamantyl)styrène sous forme d'une huile incolore.

### (d) 2-méthoxy-4-méthoxyéthoxyméthoxy-5-(1-adamantyl)phénylacétylène.

De manière analogue à l'exemple 12(d) à partir de 2 g (3,8 mmoles) du produit précédent, on obtient 1,3 g (93%) du dérivé acétylénique attendu sous forme d'une huile incolore.

### (e) 4-[2-méthoxy-5-(1-adamantyl)-4-méthoxyéthoxyméthoxy phényléthynyl]benzoate de méthyle.

De manière analogue à l'exemple 12(e) par réaction de 1,3 g (3,5 mmoles) de 2-méthoxy-4-méthoxyéthoxyméthoxy-5-(1-adamantyl)phénylacétylène avec 920 mg (3,5 mmoles) de 4-iodobenzoate de méthyle, on obtient 1,3 g (73%) de l'ester méthylique attendu de point de fusion 152-5°C.

### (f) acide 4-[2-méthoxy-5-(1-adamantyl)-4-méthoxyéthoxyméthoxyphényléthynyl] benzoïque.

De manière analogue à l'exemple 8(b) à partir de 1,3 g (2,6 mmoles) de l'ester méthylique précédent, on obtient 1,05 g (83%) d'acide 4-[2-méthoxy-5-(1-adamantyl)-4-méthoxyéthoxyméthoxyphényléthynyl] benzoïque de point de fusion 210-1°C.

### EXEMPLE 18

### Acide 4-[3-(1-adamantyl)-4-benzyloxyphenylethynyl]benzoïque.

### (a) 4-[3-(1-adamantyl)-4-benzyloxyphenylethynyl]benzoate de méthyle

De manière analogue à l'exemple 8(a) par réaction de 1,1 g (2,8 mmoles) de 4-[3-(1-adamantyl)-4-hydroxyphenylethynyl] benzoate de méthyle avec 410 µl (3,4 mmoles) de bromure de benzyle, on obtient 800 mg (57%) de l'ester méthylique attendu de point de fusion 168-9°C.

### (b) Acide 4-[3-(1-adamantyl)-4-benzyloxyphenylethynyl]benzoïque.

De manière analogue à l'exemple 8(b) à partir de 800 mg (1,68 mmole) de l'ester méthylique précédent, on obtient 690 mg (88%) d'acide 4-[3-(1-adamantyl)-4-benzyloxyphenylethynyl]benzoïque de point de fusion 288-9°C.

### EXEMPLE 19

### 4-[3-(1-adamantyl)-4-méthoxyéthoxyméthoxyphényléthynyl]benzaldéhyde.

Dans un tricol, on introduit 3 g (8,8 mmoles) de 3-(1-adamantyl)-4-méthoxy éthoxyméthoxyphénylacétylène, 1,8 g (9,7 mmoles) de 4-bromobenzaldéhyde et 50 ml de triéthylamine. On dégaze le milieu réactionnel avec de l'azote pendant 15' et ajoute 160 mg (1,7 mmole) d'acétate de palladium(ll) et 350 mg (1,3 mmole) de triphénylphosphine et agite à température ambiante pendant douze heures. On évapore à sec le milieu réactionnel, reprend par l'eau et l'éther éthylique, décante la phase organique, sèche sur sulfate de magnésium, évapore. Le résidu obtenu est purifié par chromatographie sur colonne de silice élué avec un mélange d'acétate d'éthyle et d'heptane (10-90). Aprés évaporation des solvants, on recueille 1,39 g (34%) de 4-[3-(1-adamantyl)-4-méthoxyéthoxyméthoxyphényléthynyl] benzaldéhyde de point de fusion 120-1°C.

### EXEMPLE 20

### 4-[3-(1-adamantyl)-4-méthoxyéthoxyméthoxyphényléthynyl]benzèneméthanol.

Dans un ballon, on introduit 740 mg (1,6 mmole) de 4-[3-(1-adamantyl)-4-méthoxyéthoxyméthoxyphényléthynyl]benzaldéhyde dans 40 ml d'un mélange de THF et de méthanol (50-50). A 5°C, on introduit par petites quantités 32 mg (0,8 mmole) de borohydrure de sodium et laisse remonter à température ambiante. On verse le milieu réactionnel dans l'eau, extrait avec de l'acétate d'éthyle, décante la phase organique, sèche sur sulfate de magnésium, évapore. Le résidu obtenu est purifié par chromatographie sur colonne de silice élué avec un mélange d'acétate d'éthyle et d'heptane (20-80). On recueille après évaporation des solvants, 440 mg (60%) de 4-[3-(1-adamantyl)-4-méthoxyéthoxyméthoxyphényléthynyl] benzèneméthanol de point de fusion 103-4°C.

### EXEMPLE 21

### Acétate de 4-[3-(1-adamantyl)-4-méthoxyéthoxyméthoxyphényl éthynyl]benzyle.

Dans un tricol et sous courant d'azote, on introduit 300 mg (0,7 mmole) de l'alcool obtenu à l'exemple précédent 30 ml de dichlorométhane et 230 µl (1,6 mmole) de triéthylamine. On ajoute 60 µl (0,8 mmole) de chlorure d'acétyle et agite à température ambiante pendant deux heures. On verse le milieu réactionnel dans l'eau, extrait avec du dichlorométhane, décante la phase organique, sèche sur sulfate de magnésium, évapore. Le résidu obtenu est cristallisé dans l'heptane, après filtration on recueille 190 mg (64%) d'acétate de 4-[3-(1-adamantyl)-4-méthoxyéthoxy méthoxyphényléthynyl]benzyle de point de fusion 83-4°C.

### EXEMPLE 22

### Acétate de 4-[3-(1-adamantyl)-4-methoxyethoxymethoxyphenyl ethynyl]phényle.

De manière analogue à l'exemple 12(e) par réaction de 2 g (5,9 mmoles) de 3-(1-adamantyl)-4-méthoxyéthoxyméthoxyphénylacétylène avec 1,6 g (5,9 mmoles) d'acétate de 4-bromophényle, on obtient 1,2 g (43%) de l'acétate attendu de point de fusion 92-3°C.

### EXEMPLE 23

### 4-[3-(1-adamantyl)-4-méthoxyéthoxyméthoxyphényléthynyl]phénol

De manière analogue à l'exemple 8(b) à partir de 840 mg (1,8 mmole) d'acétate de 4-[3-(1-adamantyl)-4-méthoxyéthoxyméthoxyphényléthynyl]phényle, on obtient 710 mg (92%) du dérivé phénolique attendu de point de fusion 122-3°C.

### EXEMPLE 24

### 4-[3-(1-adamantyl)-4-méthoxyéthoxyméthoxyphényléthynyl]phénoxy éthylmorpholine.

Dans un ballon on introduit 380 mg (0,9 mmole) de 4-[3-(1-adamantyl)-4-méthoxyéthoxyméthoxyphényléthynyl]phénol 200 mg de chlorhydrate de 4-(2-chloroéthyl)morpholine 300 mg (2 mmoles) de carbonate de potassium 3 mg d'iodure de potassium et 50 ml de 2-butanone. On chauffe à reflux pendant huit heures, évapore à sec le milieu réactionnel, reprend par l'eau et l'acétate d'éthyle, décante la phase organique, sèche sur sulfate de magnésium, évapore. Le résidu obtenu est trituré dans l'heptane, filtré. On recueille 330 mg (69%) de 4-[3-(1-adamantyl)-4-méthoxyéthoxyméthoxyphényléthynyl]phénoxyéthylmorpholine de point de fusion 168-9°C.

### EXEMPLE 25

### 4-[3-(1-adamantyl)-4-méthoxyéthoxyméthoxyphényléthynyl]benzamide.

### (a) chlorure de 4-[3-(1-adamantyl)-4-méthoxyéthoxyméthoxyphényléthynyl] benzoyle.

Dans un ballon, on introduit une solution de 3,14 g (6,8 mmoles) d'acide 4-[3-(1-adamantyl)-4-méthoxyéthoxyméthoxyphényléthynyl]benzoïque dans 100 ml de dichlorométhane anhydre ajoute 1,4 ml (6,8 mmoles) de dicyclohexylamine et agite pendant une heure. On ajoute ensuite 500 µl (6,8 mmoles) de chlorure de thionyle et agite une heure. On évapore à sec, reprend par l'éther éthylique anhydre, filtre le sel de dicyclohexylamine et évapore le filtrat. On recueille 3,3 g (100%) du chlorure d'acide brut qui sera utilisé tel quel pour la suite de la synthèse.

### (b) 4-[3-(1-adamantyl)-4-methoxyethoxymethoxyphenylethynyl]benzamide.

Dans un ballon, on introduit 500 µl (9,1 mmoles) d'une solution d'ammoniaque (32%) et 20 ml de THF. On ajoute goutte à goutte une solution de 1,1 g (2,3 mmoles) de chlorure de 4-[3-(1-adamantyl)-4-méthoxyéthoxyméthoxyphényléthynyl] benzoyle dissous dans 30 ml de THF et agite à température ambiante pendant trois heures. On verse le milieu réactionnel dans l'eau, extrait avec de l'acétate d'éthyle, décante la phase organique, sèche sur sulfate de magnésium, évapore. Le résidu obtenu est purifié par chromatographie sur colonne de silice élué avec un mélange de dichlorométhane et de méthanol (98-2). On recueille après évaporation des solvants 550 mg (53%) de l'amide attendu de point de fusion 206-7°C.

### EXEMPLE 26

### N-éthyl-4-[3-(1-adamantyl)-4-méthoxyéthoxyméthoxyphényléthynyl]benzamide.

De manière analogue à l'exemple 25(b) par réaction de 1,1 g (2,3 mmoles) de chlorure de 4-[3-(1-adamantyl)-4-méthoxyéthoxyméthoxyphényléthynyl]benzoyle avec 600 µl (9,1 mmoles) d'éthylamine (70%), on obtient 520 mg (47%) de l'éthylamide attendu de point de fusion 141-2°C.

### EXEMPLE 27

### Morpholide de l'acide 4-[3-(1-adamantyl)-4-methoxyethoxymethoxyphenyl ethynyl]benzoïque

De manière analogue à l'exemple 25(b) par réaction de 1,1 g (2,3 mmoles) de chlorure de 4-[3-(1-adamantyl)-4-méthoxyéthoxyméthoxyphényléthynyl]benzoyle avec 600 µl (6,8 mmoles) de morpholine, on obtient 930 mg (44%) de l'amide attendu attendu de point de fusion 142-3°C.

### EXEMPLE 28

### Acide 2-hydroxy-4-[3-(1-adamantyl)-4-methoxyethoxymethoxyphenyl ethynyl]benzoïque.

### (a) 2-hydroxy-4-[3-(1-adamantyl)-4-méthoxyéthoxyméthoxyphényléthynyl]benzoate de méthyle.

De manière analogue à l'exemple 12(e) par réaction de 690 mg (1,9 mmole) de 3-(1-adamantyl)-4-méthoxyéthoxyméthoxyphénylacétylène avec 530 mg (1,9 mmole) de 2-hydroxy-4-iodobenzoate de méthyle, on obtient 490 mg (52%) de l'ester méthylique attendu de point de fusion 96-7°C.

### (b) acide 2-hydroxy-4-[3-(1-adamantyl)-4-méthoxyéthoxyméthoxyphényl éthynyl]benzoïque.

De manière analogue à l'exemple 8(b) à partir de 500 mg (1 mmole) de 2-hydroxy-4-[3-(1-adamantyl)-4-méthoxyéthoxyméthoxyphenyléthynyl]benzoate de méthyle, on obtient 360 mg (74%) d'acide 2-hydroxy-4-[3-(1-adamantyl)-4-méthoxyéthoxyméthoxyphényléthynyl]benzoïque de point de fusion 186-7°C.

### EXEMPLE 29

### Acide 4-[3-(1-adamantyl)-4-(6-hydroxyhéxyloxy)phényléthynyl]benzoïque.

### (a) 4-[3-(1-adamantyl)-4-(6-hydroxyhéxyloxy)phényléthynyl]benzoate de méthyle.

Dans un ballon on introduit 2 g (5,1 mmoles) de 4-[3-(1-adamantyl)-4-hydroxyphényléthynyl]benzoate de méthyle 1,1 ml (8,3 mmoles) de 6-bromo-1-hexanol 1,2 g (8,8 mmoles) de carbonate de potassium 20 mg d'iodure de potassium et 100 ml de 2-butanone. On chauffe à reflux pendant douze heures, évapore à sec le milieu réactionnel, reprend par l'eau et l'acétate d'éthyle, dècante la phase organique, sèche sur sulfate de magnésium, évapore. Le résidu obtenu est purifié par chromatographie sur colonne de silice élué avec un mélange d'acétate d'éthyle et d'heptane (50-50). On recueille après évaporation des solvants 1,2 g (47%) du produit attendu de point de fusion 141-2°C.

### (b) acide 4-[3-(1-adamantyl)-4-(6-hydroxyhéxyloxy)phényléthynyl]benzoïque.

De manière analogue à l'exemple 8(b) à partir de 400 mg (0,8 mmole) de l'ester méthylique précédent, on recueille 330 mg (85%) d'acide 4-[3-(1-adamantyl)-4-(6-hydroxyhéxyloxy)phényléthynyl]benzoïque de point de fusion 228-9°C.

### EXEMPLE 30

### Acide 4-[3-(1-adamantyl)-4-(6-méthoxyhéxyloxy)phényléthynyl]benzoïque.

### (a) 4-[3-(1-adamantyl)-4-(6-méthoxyhéxyloxy)phényléthynyl]benzoate de méthyle.

Dans un ballon on introduit 390 mg (0,8 mmole) de 4-[3-(1-adamantyl)-4-(6-hydroxyhéxyloxy)phényléthynyl]benzoate de méthyle 10 ml de toluène et 200 µl d'une solution de soude à 50%. On ajoute 20 mg (5,4 mmoles) d'iodure de tétrabutylammonium, 100 µl (0,9 mmole) de sulfate de diméthyle et agite à température ambiante pendant quatre heures. On verse le milieu réactionnel dans l'eau, extrait avec de l'acétate d'éthyle, décante la phase organique, sèche sur sulfate de magnésium, évapore. Le résidu obtenu est purifié par chromatographie sur colonne de silice élué avec un mélange d'acétate d'éthyle et d'heptane (10-90). Après évaporation des solvants, on recueille 60 mg (15%) de l'ester méthylique attendu de point de fusion 112-3°C.

### (b) acide 4-[3-(1-adamantyl)-4-(6-méthoxyhéxyloxy)phényléthynyl]benzoïque.

De manière analogue à l'exemple 8(b) à partir de 170 mg (0,34 mmole) de l'ester méthylique précédent, on recueille 100 mg (61%) d'acide 4-[3-(1-adamantyl)-4-(6-méthoxyhéxyloxy)phényléthynyl]benzoïque de point de fusion 241-2°C.

### EXEMPLE 31

### Acide 4-[[3-(1-adamantyl)-4-[2-(4-morpholino)éthoxy] phényléthynyl]]benzoïque.

### (a) 4-[[3-(1-adamantyl)-4-[2-(4-morpholino)éthoxy]phényléthynyl]]benzoate de méthyle.

De manière analogue à l'exemple 29(a) par réaction de 750 mg (1,9 mmole) de 4-[3-(1-adamantyl)-4-hydroxyphényléthynyl]benzoate de méthyle avec 430 mg (2,3 mmoles) de chlorhydrate de 4-(2-chloroéthyl)morpholine, on obtient 540 mg (56%) d'ester méthylique attendu de point de fusion 208-9°C.

### (b) acide 4-[[3-(1-adamantyl)-4-[2-(4-morpholino)éthoxy] phényléthynyl]]benzoïque.

De manière analogue à l'exemple 8(b) à partir de 540 mg (1 mmole) de l'ester méthylique précédent, on recueille 410 mg (79%) d'acide 4-[[3-(1-adamantyl)-4-[2-(4-morpholino)éthoxy]phényléthynyl]]benzoïque de point de fusion 271-2°C.

### EXEMPLE 32

### Acide 4-[3-(1-adamantyl)-4-(5-carbamoylpentyloxy)phényléthynyl]benzoïque.

### (a) 4-[3-(1-adamantyl)-4-(5-carbamoylpentyloxy)phényléthynyl]benzoate de méthyle.

De manière analogue à l'exemple 29(a) par réaction de 772 mg (2 mmoles) de 4-[3-(1-adamantyl)-4-hydroxyphényléthynyl]benzoate de méthyle avec 584 mg (3 mmoles) de 6-bromohexanamide, on obtient 264 mg (26%) d'ester méthylique attendu de point de fusion 198-200°C.

### (b) acide 4-[3-(1-adamantyl)-4-(5-carbamoylpentyloxy)phényléthynyl]benzoïque.

De manière analogue à l'exemple 8(b) à partir de 254 mg (0,5 mmole) de l'ester méthylique précédent, on recueille 100 mg (40%) d'acide 4-[3-(1-adamantyl)-4-(5-carbamoylpentyloxy)phényléthynyl]benzoïque de point de fusion 269-70°C.

### EXEMPLE 33

### Acide 4-[3-(1-adamantyl)-4-(3-hydroxypropyloxy)phényléthynyl]benzoïque.

### (a) 4-[3-(1-adamantyl)-4-(3-hydroxypropyloxy)phényléthynyl]benzoate de méthyle.

De manière analogue à l'exemple 29(a) par réaction de 773 mg (2 mmoles) de 4-[3-(1-adamantyl)-4-hydroxyphényléthynyl]benzoate de méthyle avec 417 mg (3 mmoles) de 3-bromo-1-propanol, on obtient 683 mg (77%) d'ester méthylique attendu de point de fusion 180-2°C.

### (b) acide 4-[3-(1-adamantyl)-4-(3-hydroxypropyloxy)phényléthynyl]benzoïque.

De manière analogue à l'exemple 8(b) à partir de 678 mg (1,52 mmole) de l'ester méthylique précédent, on recueille 600 mg (92%) d'acide 4-[3-(1-adamantyl)-4-(3-hydroxypropyloxy)phényléthynyl]benzoïque de point de fusion 290-2°C.

### EXEMPLE 34

### Acide 4-[3-(1-adamantyl)-4-(3-hydroxy-2-méthylpropyloxy)phényléthynyl]benzoïque.

### (a) 4-[3-(1-adamantyl)-4-(3-hydroxy-2-méthylpropyloxy)phényléthynyl]benzoate de méthyle.

De manière analogue à l'exemple 29(a) par réaction de 773 mg (2 mmoles) de 4-[3-(1-adamantyl)-4-hydroxyphényléthynyl]benzoate de méthyle avec 477 mg (3 mmoles) de 3-bromo-2-méthyl-1-propanol, on obtient 250 mg (27%) d'ester méthylique attendu de point de fusion 174-6°C.

### (b) acide 4-[3-(1-adamantyl)-4-(3-hydroxy-2-méthylpropyloxy)phényléthynyl] benzoïque.

De manière analogue à l'exemple 8(b) à partir de 228 mg (0,5 mmole) de l'ester méthylique précédent, on recueille 166 mg (72%) d'acide 4-[3-(1-adamantyl)-4-(3-hydroxy-2-méthylpropyloxy)phényléthynyl]benzoïque de point de fusion 260-2°C.

### EXEMPLE 35

### Acide 4-[3-(1-adamantyl)-4-(2,2-diméthyl-1,3-dioxolanne-4-méthoxy)phényl éthynyl]benzoïque.

### (a) 4-[3-(1-adamantyl)-4-(2,2-diméthyl-1,3-dioxolanne-4-méthoxy)phényléthynyl] benzoate de méthyle.

1,22 g (3,1 mmoles) de 4-[3-(1-adamantyl)-4-hydroxyphényléthynyl]benzoate de méthyle dans 25 ml de DMF sont traités successivement par 523 mg de carbonate de potassium et 1,08 g (3,8 mmoles) de 3-tosyloxy-1,2-propanediol acétonide. Le mélange réactionnel est agité à température ambiante pendant quatre heures puis chauffé à 80°C pendant seize heures. Après traitement habituel, le résidu obtenu est trituré dans l'heptane, filtré et séché. On recueille 862 mg (55%) de l'ester méthylique attendu de point de fusion 203-4°C.

### (b) acide 4-[3-(1-adamantyl)-4-(2,2-diméthyl-1,3-dioxolanne-4-méthoxy)phényl éthynyl]benzoïque.

De manière analogue à l'exemple 8(b) à partir de 404 mg (0,8 mmole) de l'ester méthylique précédent, on recueille 341 mg (87%) d'acide 4-[3-(1-adamantyl)-4-(2,2-diméthyl-1,3-dioxolanne-4-méthoxy)phényléthynyl]benzoïque de point de fusion 272-4°C.

### EXEMPLE 36

### Acide 4-[3-(1-adamantyl)-4-(2,3-dihydroxypropyloxy)phényléthynyl]benzoïque.

### (a) 4-[3-(1-adamantyl)-4-(2,3-dihydroxypropyloxy)phényléthynyl]benzoate de méthyle.

450 mg (0,9 mmole) de 4-[3-(1-adamantyl)-4-(2,2-diméthyl-1,3-dioxolanne-4-méthoxy)phényléthynyl]benzoate de méthyle sont placés en suspension dans 10 ml d'une solution d'acide formique (40%) et chauffés à 100°C pendant trois jours. On verse le milieu réactionnel dans l'eau, extrait avec de l'acétate d'éthyle, décante la phase organique, sèche sur sulfate de magnésium, évapore. Le résidu obtenu est purifié par chromatographie sur colonne de silice élué avec du dichlorométhane. On recueille 220 mg (53%) de l'ester méthylique attendu de point de fusion 202-3°C.

### (b) acide 4-[3-(1-adamantyl)-4-(2,3-dihydroxypropyloxy)phényléthynyl]benzoïque.

De manière analogue à l'exemple 8(b) à partir de 205 mg (0,44 mmole) de l'ester méthylique précédent, on recueille 153 mg (78%) d'acide 4-[3-(1-adamantyl)-4-(2,3-dihydroxypropyloxy)phényléthynyl]benzoïque de point de fusion 300-2°C.

### EXEMPLE 37

Dans cet exemple, on a illustré diverses formulations concrètes à base des composés selon l'invention.

### A- VOIE ORALE

| (a) Comprimé de 0,2 g | |
|---|---|
| - Composé préparé à l'exemple 1 | 0,001 g |
| - Amidon | 0,114 g |
| - Phosphate bicalcique | 0,020 g |
| - Silice | 0,020 g |
| - Lactose | 0,030 g |
| - Talc | 0,010 g |
| - Stéarate de magnésium | 0,005 g |

| (b) Suspension buvable en ampoules de 10 ml | |
|---|---|
| - Composé de l'exemple 2 | 0,05 g |
| - Glycérine | 1,000g |
| - Sorbitol à 70% | 1,000g |
| - Saccharinate de sodium | 0,010 g |
| - Parahydroxybenzoate de méthyle | 0,080 g |
| - Arome qs | |
| - Eau purifiée qsp | 10 ml |

### B- VOIE TOPIQUE

| (a) Onguent | |
|---|---|
| - Composé de l'exemple 1 | 0,020 g |
| - Myristate d'isopropyle | 81,700 g |
| - Huile de vaseline fluide | 9,100 g |
| - Silice ("Aérosil 200" vendue par DEGUSSA) | 9,180 g |

| (b) Onguent | |
|---|---|
| - Composé de l'exemple 2 | 0,300 g |
| - Vaseline blanche code qsp | 100 g |

| c) Crème Eau-dans-Huile non ionique | |
|---|---|
| - Composé de l'exemple 3 | 0,100 g |
| - Mélange d'alcools de lanoline émulsifs, de cires et d'huiles ("Eucerine anhydre" vendu par BDF) | 39,900 g |
| - Parahydroxybenzoate de méthyle | 0,075 g |
| - Parahydroxybenzoate de propyle | 0,075 g |
| - Eau déminéralisée stérile qsp | 100 g |

| (d) Lotion | |
|---|---|
| - Composé de l'exemple 1 | 0,100 g |
| - Polyéthylène glycol (PEG 400) | 69,900 g |
| - Ethanol à 95% | 30,000 g |

| (e) Onguent hydrophobe | |
|---|---|
| - Composé de l'exemple 3 | 0,300 g |
| - Myristate d'isopropyle | 36,400 g |
| - Huile de silicone ("Rhodorsil 47 V 300" vendu par RHONE-POULENC) | 36,400 g |
| - Cire d'abeille | 13,600 g |
| - Huile de silicone ("Abil 300.000 cst" vendu par GOLDSCHMIDT) qsp | 100g |

| (f) Crème Huile-dans-Eau non ionique | |
|---|---|
| - Composé de l'exemple 4 | 0,500 g |
| - Alcool cétylique | 4,000 g |
| - Monostéarate de glycérole | 2,500 g |
| - Stéarate de PEG 50 | 2,500 g |
| - Beurre de karité | 9,200 g |
| - Propylène glycol | 2,000 g |
| - Parahydroxybenzoate de méthyle | 0,075 g |
| - Parahydroxybenzoate de propyle | 0,075 g |
| - Eau déminéralisée stérile qsp | 100 g |

## Revendications

1. Composés bi-aromatiques acétylénés à groupement adamantyle, caractérisés par le fait qu'ils répondent à la formule générale (I) suivante : 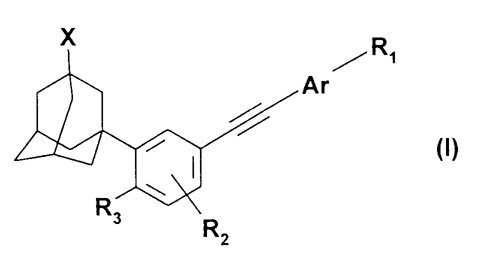 dans laquelle :
∗ X représente un atome d'hydrogène ou un atome d'halogéne,
∗ R₁ représente un atome d'hydrogène, un radical -CH₃, un radical -CH₂-O-R₄, un radical -CH₂-O-CO-R₅, un radical -O-R₆, un radical -O-(CH₂)ₘ-(CO)ₙ-R₇, un radical -(CH₂)ₚ-CO-R₈, un radical -(CH₂)ₚ-CO-O-R₉, ou encore un radical -S(O)ₚ-R₁₀, les valeurs m, n et p ainsi que les différents radicaux R₄ à R₁₀ ayant la signification donnée ci-après,
* Ar représente un radical choisi parmi les radicaux de formules (a)-(f) suivantes : 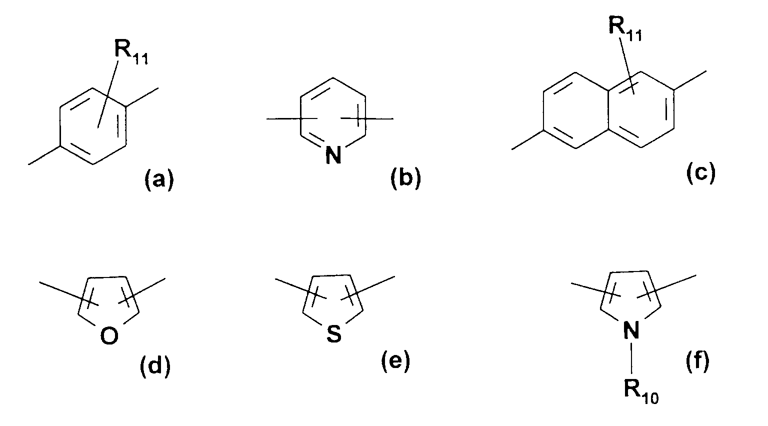 R₁₀ et R₁₁ ayant la signification donnée ci-après,
∗ R₂ représente un atome d'hydrogène ou un atome d'halogène, un radical alkyle, linéaire ou ramifié, ayant de 1 à 20 atomes de carbone, un radical -O-CH₂-O-CH₂-CH₂-O-CH₃ ou encore un radical -O-R₁₃, R₁₃ ayant la signification donnée ci-après,
∗ R₃ représente un radical -O-CH₂-O-CH₂-CH₂-O-CH₃, un radical -(Y)ₙ-(CH₂)_{q}-R₁₄, un radical (CH₂)ₘ-Y-(CH₂)_{q}-R₁₄ ou bien encore -CH=CH-(CH₂)ₜ-R₁₄, les valeurs m, n, q et t et les radicaux Y et R₁₄ ayant la signification donnée ci-après,
étant entendu que dans tout ce qui précède :
- m, identique ou différent, est un nombre entier égal à 1, 2 ou 3, n, identique ou différent, est un nombre entier égal à 0 ou 1, p est un nombre entier égal à 0, 1, 2 ou 3, q est un nombre entier compris inclusivement entre 0 et 12, et t est un nombre entier compris inclusivement entre 0 et 10,
- R₄ représente un atome d'hydrogène ou un radical alkyle en C₁-C₆,
- R₅ représente un radical alkyle en C₁-C₆,
- R₆ représente un atome d'hydrogène ou un radical alkyle en C₁-C₆,
- R₇ représente un radical alkyle en C₁-C₆ ou un hétérocycle,
- R₈ représente un atome d'hydrogène, un radical alkyle en C₁-C₆ ou un radical : 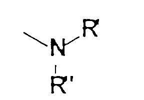 dans lequel R' et R'', identiques ou différents, représentent un atome d'hydrogène, un radical alkyle en C₁-C₆, un radical mono ou polyhydroxyalkyle, un radical aryle éventuellement substitué ou un reste d'aminoacide ou de peptide ou de sucre ou encore, pris ensemble, forment un hétérocycle,
- R₉ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- R₁₀ représente un atome d'hydrogène ou un radical alkyle en C₁-C₆,
- R₁₁ représente un atome d'hydrogène, un atome d'halogène, un radical alkyle en C₁-C₆, un radical hydroxy, un radical -O-R₁₂ ou -O-COR₁₂, R₁₂ ayant la signification donnée ci-dessous,
- R₁₂ représente un radical alkyle en C₁-C₆,
- R₁₃ représente un atome d'hydrogène ou un radical alkyle, linéaire ou ramifié, ayant de 1 à 20 atomes de carbone,
- R₁₄ représente un atome d'hydrogène, un radical alkyle en C₁-C₆, un radical alkényle, un radical alkynyle, un radical cycloaliphatique en C₃-C₆, un radical mono ou polyhydroxyalkyle dont les hydroxyles sont éventuellement protégés sous forme de méthoxy, d'acétoxy ou d'acétonide, un radical aryle ou aralkyle, un radical -CO-R₈, un radical -COOR₉, un radical S(O)ₚ-R₁₀, un radical : 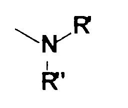 ou, mais seulement lorsque n est égal à 0 avec la signification de R₃ étant -(Y)ₙ-(CH₂)_{q}-R₁₄, un radical hydroxy, un radical -O-R₁₂, un radical -O-COR₁₂,
- Y représente un atome d'oxygène, de soufre ou un radical -S(O)ₚ.
ainsi que les sels et les analogues chiraux desdits composés de formule (I).

2. Composés selon la revendication 1, caractérisés en ce qu'ils se présentent sous forme de sels d'un métal alcalin ou alcalino-terreux, ou encore de zinc ou d'une amine organique.

3. Composés selon l'une des revendications 1 ou 2, caractérisés en ce que les radicaux alkyles en C₁-C₆ sont choisis dans le groupe constitué par les radicaux méthyle, éthyle, isopropyle, butyle, tertiobutyle et hexyle.

4. Composés selon l'une quelconque des revendications précédentes, caractérisés en ce que les radicaux alkyles linéaires ou ramifiés ayant de 1 à 20 atomes de carbone sont choisis dans le groupe constitué par les radicaux méthyle, éthyle, propyle, 2-éthyl-hexyle, octyle, dodécyle, hexadécyle et octadécyle.

5. Composés selon l'une quelconque des revendications précédentes, caractérisés en ce que les radicaux monohydroxyalkyles sont choisis dans le groupe constitué par les radicaux 2-hydroxyéthyle, 2-hydroxypropyle ou 3-hydroxypropyle.

6. Composés selon l'une quelconque des revendications précédentes, caractérisés en ce que les radicaux polyhydroxyalkyles sont choisis dans le groupe constitué par les radicaux 2,3-dihydroxypropyle, 2,3,4-trihydroxybutyle, 2,3,4,5-tétrahydroxypentyle ou le reste du pentaérythritol.

7. Composés selon l'une quelconque des revendications précédentes, caractérisés en ce que le radical aryle est un radical phényle éventuellement substitué par au moins un atome d'halogène, un hydroxyle ou une fonction nitro.

8. Composés selon l'une quelconque des revendications précédentes caractérisés en ce que les radicaux aralkyles sont choisis dans le groupe constitué par les radicaux benzyle ou phénéthyle éventuellement substitués par au moins un atome d'halogène, un hydroxyle ou une fonction nitro.

9. Composés selon l'une quelconque des revendications précédentes, caractérisés en ce que les radicaux alkényles sont choisis dans le groupe constitué par les radicaux contenant de 2 à 5 atomes de carbone et présentant une ou plusieurs insaturations éthyléniques, en particulier le radical allyle.

10. Composés selon l'une quelconque des revendications précédentes, caractérisés en ce que les radicaux alkynyles sont choisis dans le groupe constitué par les radicaux contenant de 2 à 6 atomes de carbone, en particulier le radical propargyle.

11. Composés selon l'une quelconque des revendications précédentes, caractérisés en ce que les restes de sucre sont choisis dans le groupe constitué par les restes de glucose, de galactose, de mannose ou d'acide glucuronique.

12. Composés selon l'une quelconque des revendications précédentes, caractérisés en ce que les restes d'aminoacide sont choisis dans le groupe constitué par les restes dérivant de la lysine, de la glycine ou de l'acide aspartique.

13. Composés selon l'une quelconque des revendications précédentes, caractérisés en ce que les restes de peptides sont choisis dans le groupe constitué par les restes de dipeptides ou de tripeptides.

14. Composés selon l'une quelconque des revendications précédentes, caractérisés en ce que les radicaux hétérocycliques sont choisis dans le groupe constitué par les radicaux pipéridino, morpholino, pyrrolidino ou pipérazino, éventuellement substitués en position 4 par un radical alkyle en C₁-C₆ ou mono ou polyhydroxyalkyle.

15. Composés selon l'une quelconque des revendications précédentes, caractérisés en ce que les atomes d'halogène sont choisis dans le groupe constitué par le fluor, le chlore et le brome.

16. Composés selon la revendication 1, caractérisés par le fait qu'ils sont pris, seuls ou en mélanges, dans le groupe constitué par :
Acide 4-[3-(1-adamantyl)-4-methoxyphenylethynyl]benzoïque
Acide 4-[3-(1-adamantyl)-4 methoxyethoxymethoxyphenylethynyl]benzoïque
Acide 4-[3-(1-adamantyl)-4-hydroxyphenylethynyl]benzoïque
Acide 5-[3-(1-adamantyl)-4-methoxyphenylethynyl]-2-thiophenecarboxylique
Acide 5-[3-(1-adamantyl)-4-hydroxyphenylethynyl]-2-thiophenecarboxylique
Acide 2-[3-(1-adamantyl)-4-methoxyphenylethynyl]-4-thiophenecarboxylique
Acide 6-[3-(1-adamantyl)-4-methoxyphenylethynyl]-2-naphtoïque
Acide 4-[3-(1-adamantyl)-4-nonyloxyphényléthynyl]benzoïque
Acide 4-[3-(1-adamantyl)-4-héxyloxyphényléthynyl]benzoïque
Acide 4-[3-(1-adamantyl)-4-dodécyloxyphényléthynyl]benzoïque
Acide 4-[3-(1-adamantyl)-4-cyclopropylméthoxyphényléthynyl]benzoïque
Acide 4-[2-héxyloxy-5-(1-adamantyl)-4-héxyloxyphényléthynyl]benzoïque
Acide 4-[3-(1-adamantyl)-4-heptyloxyphényléthynyl]benzoïque
Acide 6-[3-(1-adamantyl)-4-methoxyethoxymethoxyphenylethynyl] nicotinique
Acide 4-[2-methoxy-5-(1-adamantyl)-4-methoxyphenylethynyl]benzoïque
Acide 4-[2-méthoxyéthoxyméthoxy-5-(1-adamantyl)-4-méthoxyéthoxy méthoxyphenyléthynyl]benzoïque
Acide 4-[2-methoxy-5-(1-adamantyl)-4-methoxyethoxymethoxyphenyl ethynyl]benzoïque
Acide 4-[3-(1-adamantyl)-4-benzyloxyphenylethynyl]benzoïque
4-[3-(1-adamantyl)-4-méthoxyéthoxyméthoxyphényléthynyl]benzaldéhyde
4-[3-(1-adamantyl)-4-méthoxyéthoxyméthoxyphényléthynyl] benzèneméthanol
Acétate de 4-[3-(1-adamantyl)-4-méthoxyéthoxyméthoxyphényléthynyl] benzyle
Acétate de 4-[3-(1-adamantyl)-4-methoxyethoxymethoxyphenylethynyl] phényle
4-[3-(1-adamantyl)-4-méthoxyéthoxyméthoxyphényléthynyl]phénol
4-[3-(1-adamantyl)-4-méthoxyéthoxyméthoxyphényléthynyl]phénoxy éthylmorpholine
4-[3-(1-adamantyl)-4-méthoxyéthoxyméthoxyphényléthynyl]benzamide
N-éthyl-4-[3-(1-adamantyl)-4-méthoxyéthoxyméthoxyphényléthynyl] benzamide
Morpholide de l'acide 4-[3-(1-adamantyl)-4-methoxyethoxymethoxyphenyl ethynyl]benzoïque
Acide 2-hydroxy-4-[3-(1 -adamantyl)-4-methoxyethoxymethoxyphenyl ethynyl]benzoïque
Acide 4-[3-(1-adamantyl)-4-(6-hydroxyhéxyloxy)phényléthynyl]benzoïque
Acide 4-[3-(1-adamantyl)-4-(6-méthoxyhéxyloxy)phényléthynyl]benzoïque
Acide 4-[[3-(1-adamantyl)-4-[2-(4-morpholino)éthoxy]phényléthynyl]] benzoïque
Acide 4-[3-(1-adamantyl)-4-(3-carbamoylpropyloxy)phényléthynyl] benzoïque
Acide 4-[3-(1-adamantyl)-4-(5-carbamoylpentyloxy)phényléthynyl] benzoïque
Acide 4-[3-(1-adamantyl)-4-(3-hydroxypropyloxy)phényléthynyl]benzoïque
Acide 4-[3-(1-adamantyl)-4-(3-hydroxy-2-méthylpropyloxy)phényléthynyl] benzoïque
Acide 4-[3-(1-adamantyl)-4-(2,2-diméthyl-1,3-dioxolanne-4-méthoxy)phényl éthynyl]benzoïque
Acide 4-[3-(1-adamantyl)-4-(2,3-dihydroxypropyloxy)phényléthynyl] benzoïque
Acide 4-[3-(1-adamantyl)-4-méthoxyéthoxyéthylphényléthynyl]benzoïque
Acide 4-[3-(1-adamantyl)-4-méthoxyméthoxypropylphényléthynyl] benzoïque
Acide 4-[3-(1-adamantyl)-4-méthoxyéthoxypropylphényléthynyl]benzoïque
Acide 4-[3-(1-adamantyl)-4-acétoxybutoxyphényléthynyl]benzoïque
Acide 4-[3-(1-adamantyl)-4-acétoxypropyloxyphényléthynyl]benzoïque.

17. Composés selon la revendication 1, caractérisés par le fait qu'ils présentent l'une au moins, et de préférence toutes, les caractéristiques suivantes : R₁ est un radical -(CH₂)ₚ-CO-R₈ ; R₃ est un radical -O-CH₂-O-CH₂-CH₂-O-CH₃ ou -(Y)ₙ-(CH₂)_{q}-R₁₄ ; Ar est un radical choisi parmi les radicaux de formule (a) et (e) ; et R₂ est un radical alkyle, linéaire ou ramifié, ayant de 1 à 20 atomes de carbone ou encore un radical -O-R₁₃.

18. Composés selon l'une quelconque des revendications précédentes pour une utilisation comme médicament.

19. Composés selon la revendication 18 pour une utilisation comme médicament destiné au traitement des affections dermatologiques, rhumatismales, respiratoires, cardiovasculaires et ophtalmologiques, ainsi qu'au traitement ou à la prévention de l'ostéoporose.

20. Utilisation de l'un au moins des composés définis aux revendications 1 à 17 pour la fabrication d'un médicament destiné au traitement des affections dermatologiques, rhumatismales, respiratoires, cardiovasculaires et ophtalmologiques, ainsi qu'au traitement ou à la prévention de l'ostéoporose.

21. Composition pharmaceutique, caractérisée par le fait qu'elle comprend, dans un support pharmaceutiquement acceptable, au moins l'un des composés tels que définis à l'une quelconque des revendications 1 à 17.

22. Composition selon la revendication 21, caractérisée en ce que la concentration en composé(s) selon l'une des revendication 1 à 17 est comprise entre 0,001 % et 5 % en poids par rapport à l'ensemble de la composition.

23. Composition cosmétique, caractérisée par le fait qu'elle comprend, dans un support cosmétiquement acceptable, au moins l'un des composés tels que définis à l'une quelconque des revendications 1 à 17.

24. Composition selon la revendication 23, caractérisée en ce que la concentration en composé(s) selon l'une des revendications 1 à 17 est comprise entre 0,001 % et 3 % en poids par rapport à l'ensemble de la composition.

25. Utilisation d'une composition cosmétique telle que définie à l'une des revendications 23 ou 24 pour l'hygiène corporelle ou capillaire.

## Claims

1. Biaromatic acetylene compounds containing an adamantyl group, characterized in that they correspond to the following general formula (I): 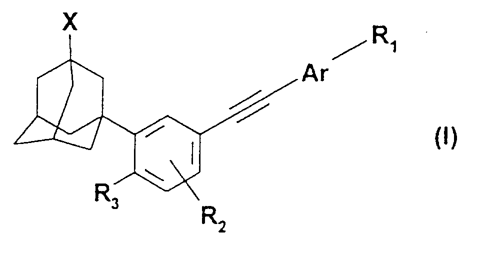 in which:
* X represents a hydrogen atom or a halogen atom,
* R₁ represents a hydrogen atom, a -CH₃ radical, a -CH₂-O-R₄ radical, a -CH₂-O-CO-R₅ radical, an -O-R₆ radical, an -O-(CH₂)ₘ-(CO)ₙ-R₇ radical, a -(CH₂)ₚ-CO-R₈ radical, a -(CH₂)ₚ-CO-O-R₉ radical or alternatively an -S(O)ₚ-R₁₀ radical, the m, n and p values and the various R₄ to R₁₀ radicals having the meaning given hereinbelow,
* Ar represents a radical chosen from the following radicals of formulae (a)-(f): 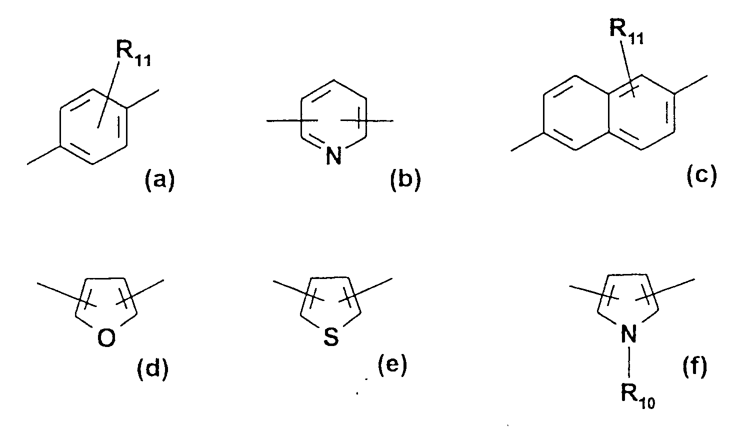 R₁₀ and R₁₁ having the meaning given hereinbelow,
* R₂ represents a hydrogen atom or a halogen atom, a linear or branched alkyl radical having from 1 to 20 carbon atoms, an -O-CH₂-O-CH₂-CH₂-O-CH₃ radical or alternatively an -O-R₁₃ radical, R₁₃ having the meaning given hereinbelow,
* R₃ represents an -O-CH₂-O-CH₂-CH₂-O-CH₃ radical, a -(Y)ₙ-(CH₂)_{q}-R₁₄ radical, a -(CH₂)ₘ-Y-(CH₂)_{q}-R₁₄ radical or alternatively -CH=CH-(CH₂)ₜ-R₁₄, the m, n, q and t values and the Y and R₁₄ radicals having the meaning given hereinbelow,
it being understood that in all the above:
- m, which is identical or different, is an integer equal to 1, 2 or 3, n, which is identical or different, is an integer equal to 0 or 1, p is an integer equal to 0, 1, 2 or 3, q is an integer between 0 and 12 inclusive and t is an integer between 0 and 10 inclusive,
- R₄ represents a hydrogen atom or a C₁-C₆ alkyl radical,
- R₅ represents a C₁-C₆ alkyl radical,
- R₆ represents a hydrogen atom or a C₁-C₆ alkyl radical,
- R₇ represents a C₁-C₆ alkyl radical or a heterocycle,
- R₈ represents a hydrogen atom, a C₁-C₆ alkyl radical or a radical: 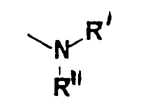 in which R' and R'', which are identical or different, represent a hydrogen atom, a C₁-C₆ alkyl radical, a mono- or polyhydroxyalkyl radical, an optionally substituted aryl radical or an amino acid or peptide or sugar residue or alternatively, taken together, form a heterocycle,
- R₉ represents a hydrogen atom, a linear or branched alkyl radical having from 1 to 20 carbon atoms, an alkenyl radical, a mono- or polyhydroxyalkyl radical, an optionally substituted aryl or aralkyl radical or a sugar residue or an amino acid or peptide residue,
- R₁₀ represents a hydrogen atom or a C₁-C₆ alkyl radical,
- R₁₁ represents a hydrogen atom, a halogen atom, a C₁-C₆ alkyl radical, a hydroxyl radical, or an -O-R₁₂ or -O-COR₁₂ radical, R₁₂ having the meaning given hereinbelow,
- R₁₂ represents a C₁-C₆ alkyl radical,
- R₁₃ represents a hydrogen atom or a linear or branched alkyl radical having from 1 to 20 carbon atoms,
- R₁₄ represents a hydrogen atom, a C₁-C₆ alkyl radical, an alkenyl radical, an alkynyl radical, a C₃-C₆ cycloaliphatic radical, a mono- or polyhydroxyalkyl radical in which the hydroxyls are optionally protected in the methoxy, acetoxy or acetonide form, an aryl or aralkyl radical, a -CO-R₈ radical, a -COOR₉ radical, an -S(O)ₚ-R₁₀ radical, a radical: 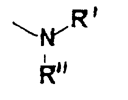 or, but only when n is equal to 0 with the meaning of R₃ being -(Y)ₙ-(CH₂)_{q}-R₁₄, a hydroxyl radical, an -O-R₁₂ radical or an -O-COR₁₂ radical,
- Y represents an oxygen or sulphur atom or an -S(O)ₚ radical,
and the salts and the chiral analogues of the said compounds of formula (I).

2. Compounds according to Claim 1, characterized in that they are provided in the form of salts of an alkali metal or alkaline-earth metal or alternatively of zinc or of an organic amine.

3. Compounds according to either of Claims 1 and 2, characterized in that the C₁-C₆ alkyl radicals are chosen from the group comprising methyl, ethyl, isopropyl, butyl, tert-butyl and hexyl radicals.

4. Compounds according to any one of the preceding claims, characterized in that the linear or branched alkyl radicals having from 1 to 20 carbon atoms are chosen from the group comprising the methyl, ethyl, propyl, 2-ethylhexyl, octyl, dodecyl, hexadecyl and octadecyl radicals.

5. Compounds according to any one of the preceding claims, characterized in that the monohydroxyalkyl radicals are chosen from the group comprising the 2-hydroxyethyl, 2-hydroxypropyl or 3-hydroxypropyl radicals.

6. Compounds according to any one of the preceding claims, characterized in that the polyhydroxyalkyl radicals are chosen from the group comprising the 2,3-dihydroxypropyl, 2,3,4-trihydroxybutyl or 2,3,4,5-tetrahydroxypentyl radicals or the pentaerythritol residue.

7. Compounds according to any one of the preceding claims, characterized in that the aryl radical is a phenyl radical optionally substituted by at least one halogen atom, one hydroxyl or one nitro functional group.

8. Compounds according to any one of the preceding claims, characterized in that the aralkyl radicals are chosen from the group comprising the benzyl or phenethyl radicals optionally substituted by at least one halogen atom, one hydroxyl or one nitro functional group.

9. Compounds according to any one of the preceding claims, characterized in that the alkenyl radicals are chosen from the group comprising the radicals containing from 2 to 5 carbon atoms and having one or a number of ethylenic unsaturations and in particular the allyl radical.

10. Compounds according to any one of the preceding claims, characterized in that the alkynyl radicals are chosen from the group comprising the radicals containing from 2 to 6 carbon atoms and in particular the propargyl radical.

11. Compounds according to any one of the preceding claims, characterized in that the sugar residues are chosen from the group comprising the glucose, galactose, mannose or glucuronic acid residues.

12. Compounds according to any one of the preceding claims, characterized in that the amino acid residues are chosen from the group comprising the residues deriving from lysine, glycine or aspartic acid.

13. Compounds according to any one of the preceding claims, characterized in that the peptide residues are chosen from the group comprising the dipeptide or tripeptide residues.

14. Compounds according to any one of the preceding claims, characterized in that the heterocyclic radicals are chosen from the group comprising the piperidino, morpholino, pyrrolidino or piperazino radicals, optionally substituted in the 4-position by a C₁-C₆ alkyl or mono- or polyhydroxyalkyl radical.

15. Compounds according to any one of the preceding claims, characterized in that the halogen atoms are chosen from the group comprising fluorine, chlorine and bromine.

16. Compounds according to Claim 1, characterized in that they are taken, alone or as mixtures, from the group comprising:
4-[3-(1-Adamantyl)-4-methoxyphenylethynyl]benzoic acid
4-[3-(1-Adamantyl)-4-methoxyethoxymethoxyphenylethynyl]benzoic acid
4-[3-(1-Adamantyl)-4-hydroxyphenylethynyl]benzoic acid
5-[3-(1-Adamantyl)-4-methoxyphenylethynyl]-2-thiophenecarboxylic acid
5-[3-(1-Adamantyl)-4-hydroxyphenylethynyl]-2-thiophenecarboxylic acid
2-[3-(1-Adamantyl)-4-methoxyphenylethynyl]-4-thiophenecarboxylic acid
6-[3-(1-Adamantyl)-4-methoxyphenylethynyl]-2-naphthoic acid
4-[3-(1-Adamantyl)-4-nonyloxyphenylethynyl]benzoic acid
4-[3-(1-Adamantyl)-4-hexyloxyphenylethynyl]benzoic acid
4-[3-(1-Adamantyl)-4-dodecyloxyphenylethynyl]benzoic acid
4-[3-(1-Adamantyl)-4-cyclopropylmethoxyphenylethynyl]benzoic acid
4-[2-Hexyloxy-5-(1-adamantyl)-4-hexyloxyphenylethynyl]benzoic acid
4-[3-(1-Adamantyl)-4-heptyloxyphenylethynyl]benzoic acid
6-[3-(1-Adamantyl)-4-methoxyethoxymethoxyphenylethynyl]nicotinic acid
4-[2-Methoxy-5-(1-adamantyl)-4-methoxyphenylethynyl]benzoic acid
4-[2-Methoxyethoxymethoxy-5-(1-adamantyl)-4-methoxyethoxymethoxyphenylethynyl]benzoic acid
4-[2-Methoxy-5-(1-adamantyl)-4-methoxyethoxymethoxyphenylethynyl]benzoic acid
4-[3-(1-Adamantyl)-4-benzyloxyphenylethynyl]benzoic acid
4-[3-(1-Adamantyl)-4-methoxyethoxymethoxyphenylethynyl]benzaldehyde
4-[3-(1-Adamantyl)-4-methoxyethoxymethoxyphenylethynyl]benzenemethanol
4-[3-(1-Adamantyl)-4-methoxyethoxymethoxyphenylethynyl]benzyl acetate
4-[3-(1-Adamantyl)-4-methoxyethoxymethoxyphenylethynyl]phenyl acetate
4-[3-(1-Adamantyl)-4-methoxyethoxymethoxyphenylethynyl]phenol
4-[3-(1-Adamantyl)-4-methoxyethoxymethoxyphenylethynyl]phenoxyethylmorpho-line
4-[3-(1-Adamantyl)-4-methoxyethoxymethoxyphenylethynyl]benzamide
N-Ethyl-4-[3-(1-adamantyl)-4-methoxyethoxymethoxyphenylethynyl]benzamide
4-[3-(1-Adamantyl)-4-methoxyethoxymethoxyphenylethynyl]benzoic acid morpholide
2-Hydroxy-4-[3-(1-adamantyl)-4-methoxyethoxymethoxyphenylethynyl]benzoic acid
4-[3-(1-Adamantyl)-4-(6-hydroxyhexyloxy)phenylethynyl]benzoic acid
4-[3-(1-Adamantyl)-4-(6-methoxyhexyloxy)phenylethynyl]benzoic acid
4-[3-(1-Adamantyl)-4-[2-(4-morpholino)-ethoxy]-phenylethynyl]benzoic acid
4-[3-(1-Adamantyl)-4-(3-carbamoylpropyloxy)phenylethynyl]benzoic acid
4-[3-(1-Adamantyl)-4-(5-carbamoylpentyloxy)phenylethynyl]benzoic acid
4-[3-(1-Adamantyl)-4-(3-hydroxypropyloxy)phenylethynyl]benzoic acid
4-[3-(1-Adamantyl)-4-(3-hydroxy-2-methylpropyloxy)phenylethynyl]benzoic acid
4-[3-(1-Adamantyl)-4-[(2,2-dimethyl-1,3-dioxolan-4-yl)methoxy]phenylethynyl]benzoic acid
4-[3-(1-Adamantyl)-4-(2,3-dihydroxypropyloxy)phenylethynyl]benzoic acid
4-[3-(1-Adamantyl)-4-methoxyethoxyethylphenylethynyl]benzoic acid
4-[3-(1-Adamantyl)-4-methoxymethoxypropylphenylethynyl]benzoic acid
4-[3-(1-Adamantyl)-4-methoxyethoxypropylphenylethynyl]benzoic acid
4-[3-(1-Adamantyl)-4-acetoxybutoxyphenylethynyl]benzoic acid
4-[3-(1-Adamantyl)-4-acetoxypropyloxyphenylethynyl]benzoic acid.

17. Compounds according to Claim 1, characterized in that they have at least one, and preferably all, of the following characteristics: R₁ is a -(CH₂)ₚ-CO-R₈ radical; R₃ is an -O-CH₂-O-CH₂-CH₂-O-CH₃ or -(Y)ₙ-(CH₂)_{q}-R₁₄ radical; Ar is a radical chosen from the radicals of formula (a) and (e); and R₂ is a linear or branched alkyl radical having from 1 to 20 carbon atoms or alternatively an -O-R₁₃ radical.

18. Compounds according to any one of the preceding claims for use as medicament.

19. Compounds according to Claim 18 for use as medicament intended for the treatment of dermatological, rheumatic, respiratory, cardiovascular and ophthalmological conditions, as well as for the treatment or prevention of osteoporosis.

20. Use of at least one of the compounds defined in Claims 1 to 17 for the manufacture of a medicament intended for the treatment of dermatological, rheumatic, respiratory, cardiovascular and ophthalmological conditions, as well as for the treatment or prevention of osteoporosis.

21. Pharmaceutical composition, characterized in that it comprises, in a pharmaceutically acceptable vehicle, at least one of the compounds as defined in any one of Claims 1 to 17.

22. Composition according to Claim 21, characterized in that the concentration of compound(s) according to one of Claims 1 to 17 is between 0.001% and 5% by weight with respect to the whole of the composition.

23. Cosmetic composition, characterized in that it comprises, in a cosmetically acceptable vehicle, at least one of the compounds as defined in any one of Claims 1 to 17.

24. Composition according to Claim 23, characterized in that the concentration of compound(s) according to one of Claims 1 to 17 is between 0.001% and 3% by weight with respect to the whole of the composition.

25. Use of a cosmetic composition as defined in either of Claims 23 and 24 for body or hair hygiene.

## Patentansprüche

1. Biaromatische Acetylenverbindungen mit Adamantylgruppe,
**dadurch gekennzeichnet, daß**
sie der folgenden allgemeinen Formel (I) entsprechen: 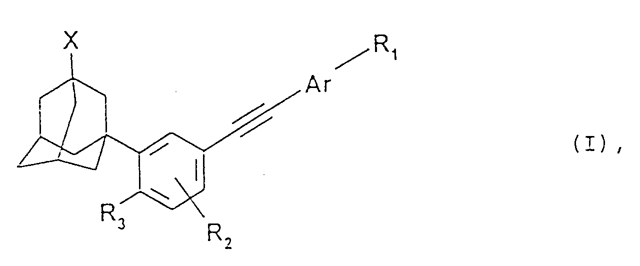 worin bedeuten:
• X Wasserstoff oder Halogen,
• R₁ Wasserstoff, eine Gruppe -CH₃, eine Gruppe -CH₂-O-R₄, eine Gruppe -CH₂-Q-CO-R₅, eine Gruppe -O-R₆, eine Gruppe -O-(CH₂)ₘ-(CO)ₙR₇, eine Gruppe -(CH₂)ₚ-CO-R₈, eine Gruppe -(CH₂)ₚ-CO-O-R₉ oder auch eine Gruppe -S(O)ₚ-R₁₀, wobei die Werte m, n und p sowie die verschiedenen Gruppen R₄ bis R₁₀ die nachstehend angegebenen Bedeutungen aufweisen,
• Ar eine Gruppe, die unter den Gruppen der folgenden Formeln (a) bis (f) ausgewählt ist: 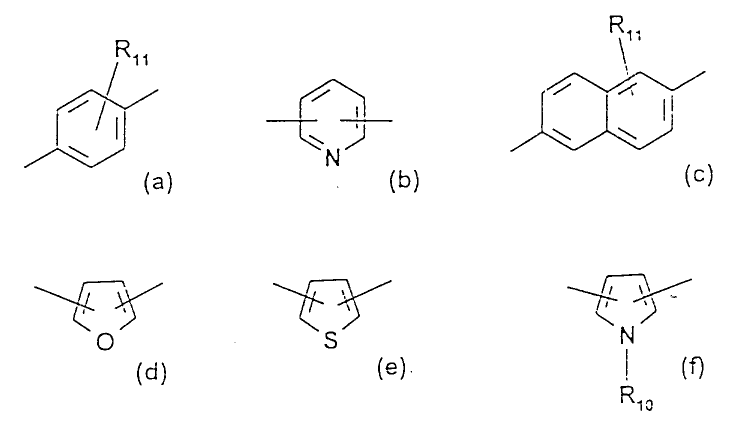 wobei R₁₀ und R₁₁ die nachfolgend angegebene Bedeutung aufweisen,
• R₂ ein Wasserstoff- oder Halogenatom, eine geradkettige oder verzweigte Alkylgruppe mit 1 bis 20 Kohlenstoffatomen, eine Gruppe -O-CH₂-O-CH₂-CH₂-O-CH₃ oder eine Gruppe -O-R₁₃, wobei R₁₃ die nachfolgend angegebene Bedeutung aufweist,
• R₃ eine Gruppe -O-CH₂-O-CH₂-CH₂-O-CH₃, eine Gruppe -(Y)ₙ₋(CH₂)_{q}-R₁₄, eine Gruppe -(CH₂)ₘ-Y-(CH₂)_{q}-R₁₄ oder auch -CH=CH-(CH₂)ₜ-R₁₄, wobei die Werte m, n, q und t und die Gruppen Y und R₁₄ die unten angegebenen Bedeutungen aufweisen,
mit der Maßgabe, daß im folgenden bedeuten:
- die Buchstaben m, die identisch oder voneinander verschieden sind, eine ganze Zahl 1, 2 oder 3, die Buchstaben n, die identisch oder voneinander verschieden sind, Null oder 1, p Null, 1, 2 oder 3, q Null oder eine ganze Zahl im Bereich von 1 bis einschließlich 12 und t Null oder eine ganze Zahl von 1 bis einschließlich 10,
- R₄ Wasserstoff oder eine C₁₋₆-Alkylgruppe,
- R₅ eine C₁₋₆-Alkylgruppe,
- R₆ Wasserstoff oder eine C₁₋₆-Alkylgruppe,
- R₇ eine C₁₋₆-Alkylgruppe oder einen Heterocyclus,
- R₈ ein Wasserstoffatom, eine C₁₋₆-Alkylgruppe, oder eine Gruppe: 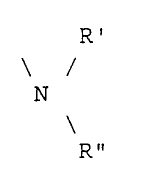 worin R' und R'', die identisch oder voneinander verschieden sind, Wasserstoff, eine C₁₋₆-Alkylgruppe, Mono- oder Polyhydroxyalkylgruppe, Arylgruppe, die gegebenenfalls substituiert ist, oder einen Aminosäure-, Petid- oder Zuckerrest bedeuten oder gemeinsam einen Heterocyclus bilden,
- R₉ Wasserstoff, eine geradkettige oder verzweigte Alkylgruppe mit 1 bis 20 Kohlenstoffatomen, Alkenylgruppe, Mono- oder Polyhydroxyalkylgruppe, Aryl- oder Aralkylgruppe, die gegebenenfalls substituiert sind, oder einen Zucker-, Aminosäure- oder Peptidrest,
- R₁₀ Wasserstoff oder eine C₁₋₆-Alkylgruppe,
- R₁₁ Wasserstoff, Halogen, eine C₁₋₆-Alkylgruppe, Hydroxy, eine Gruppe -O-R₁₂ oder -O-COR₁₂, wobei R₁₂ die nachfolgend angegebene Bedeutung aufweist,
- R₁₂ eine C₁₋₆-Alkylgruppe,
- R₁₃ Wasserstoff oder eine geradkettige oder verzweigte Alkylgruppe mit 1 bis 20 Kohlenstoffatomen,
- R₁₄ Wasserstoff, eine C₁₋₆-Alkylgruppe, Alkenyl, Alkinyl, eine cycloaliphatische Gruppe mit 3 bis 6 Kohlenstoffatomen, Mono- oder Polyhydroxyalkyl, wobei die Hydroxygruppen gegebenenfalls in Form von Methoxy, Acetoxy oder Acetonid geschützt sind, Aryl oder Aralkyl, eine Gruppe -CO-R₈, eine Gruppe -COOR₉, eine Gruppe -S(O)ₚ-R₁₀, eine Gruppe: 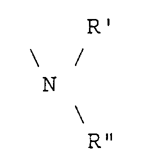 oder, falls n Null ist und R₃ die Gruppe -(Y)ₙ-(CH₂)_{q}-R₁₄ bedeutet, Hydroxy, eine Gruppe -O-R₁₂ und eine Gruppe -O-COR₁₂,
- Y Sauerstoff, Schwefel oder eine Gruppe -S(O)ₚ
sowie die Salze und chiralen Analoga der Verbindungen der Formel (I).

2. Verbindungen nach Anspruch 1, dadurch gekennzeichnet, daß sie in Form von Alkali- oder Erdalkalisalzen oder auch Zinksalzen oder Salzen anorganischer Amine vorliegen.

3. Verbindungen nach einem der Ansprüche 1 oder 2, dadurch gekennzeichnet, daß die C₁₋₆-Alkylgruppen unter den Gruppen Methyl, Ethyl, Isopropyl, Butyl, *tert*.-Butyl und Hexyl ausgewählt sind.

4. Verbindungen nach einem der vorhergehenden Ansprüche, dadurch gekennzeichnet, daß die geradkettigen oder verzweigten Alkylgruppen mit 1 bis 20 Kohlenstoffatomen unter den Gruppen Methyl, Ethyl, Propyl, 2-Ethylhexyl, Octyl, Dodecyl, Hexadecyl und Octadecyl ausgewählt sind.

5. Verbindungen nach einem der vorhergehenden Ansprüche, dadurch gekennzeichnet, daß die Monohydroxyalkylgruppen unter den Gruppen 2-Hydroxyethyl, 2-Hydroxypropyl oder 3-Hydroxypropyl ausgewählt sind.

6. Verbindungen nach einem der vorhergehenden Ansprüche, dadurch gekennzeichnet, daß die Polyhydroxyalkylgruppen unter den Gruppen 2,3-Dihydroxypropyl, 2,3,4-Trihydroxybutyl, 2,3,4,5-Tetrahydroxypentyl oder Pentaerythrit ausgewählt sind.

7. Verbindungen nach einem der vorhergehenden Ansprüche, dadurch gekennzeichnet, daß die Arylgruppe eine Phenylgruppe ist, die gegebenenfalls mit mindestens einem Halogenatom, einer Hydroxygruppe oder einer Nitrogruppe substituiert ist.

8. Verbindungen nach einem der vorhergehenden Ansprüche, dadurch gekennzeichnet, daß die Aralkylgruppen unter den Gruppen Benzyl oder Phenylethyl ausgewählt ist, die gegebenenfalls mit mindestens einem Halogenatom, einer Hydroxygruppe oder einer Nitrogruppe substituiert sind.

9. Verbindungen nach einem der vorhergehenden Ansprüche, dadurch gekennzeichnet, daß die Alkenylgruppen unter den Gruppen mit 2 bis 5 Kohlenstoffatomen, die eine oder mehrere ethylenische Doppelbindungen aufweisen, und insbesondere der Allylgruppe ausgewählt sind.

10. Verbindungen nach einem der vorhergehenden Ansprüche, dadurch gekennzeichnet, daß die Alkinylgruppen unter den Gruppen mit 2 bis 6 Kohlenstoffatomen und insbesondere der Propargylgruppe ausgewählt sind.

11. Verbindungen nach einem der vorhergehenden Ansprüche, dadurch gekennzeichnet, daß die Zuckerreste unter den Gruppen Glucose, Galactose, Mannose und Glucuronsäure ausgewählt sind.

12. Verbindungen nach einem der vorhergehenden Ansprüche, dadurch gekennzeichnet, daß die Aminosäurereste unter den von Lysin, Glycin oder Asparaginsäure abgeleiteten Gruppen ausgewählt sind.

13. Verbindungen nach einem der vorhergehenden Ansprüche, dadurch gekennzeichnet, daß die Peptidreste unter den Dipeptid- oder Tripeptidgruppen ausgewählt sind.

14. Verbindungen nach einem der vorhergehenden Ansprüche, dadurch gekennzeichnet, daß die heterocyclischen Gruppen unter den Gruppen Piperidino, Morpholino, Pyrrolidino oder Piperazino ausgewählt sind, die gegebenenfalls in 4-Stellung mit einer C₁₋₆-Alkylgruppe oder Mono- oder Polyhydroxyalkylgruppe substituiert sind.

15. Verbindungen nach einem der vorhergehenden Ansprüche, dadurch gekennzeichnet, daß die Halogenatome unter Fluor, Chlor und Brom ausgewählt sind.

16. Verbindungen nach Anspruch 1, dadurch gekennzeichnet, daß sie einzeln oder im Gemisch aus folgender Gruppe ausgewählt sind:
4-[3-(1-Adamantyl)-4-methoxyphenylethinyl]-benzoesäure
4-[3-(1-Adamantyl)-4-methoxyethoxymethoxyphenylethinyl]-benzoesäure
4-[3-(1-Adamantyl)-4-hydroxyphenylethinyl]-benzoesäure
5-[3-(1-Adamantyl)-4-methoxyphenylethinyl]-2-thiophencarbonsäure
5-[3-1-Adamantyl)-4-hydroxyphenylethinyl]-2-thiophencarbonsäure
2-[3-(1-Adamantyl)-4-methoxyphenylethinyl]-4-thiophencarbonsäure
6-[3-(1-Adamantyl)-4-methoxyphenylethinyl]-2-naphthoesäure
4-[3-(1-Adamantyl)-4-nonyloxyphenylethinyl]-benzoesäure
4-[3-(1-Adamantyl)-4-hexyloxyphenylethinyl]-benzoesäure
4-[3-(1-Adamantyl)-4-dodecyloxyphenylethinyl]-benzoesäure
4-[3-(1-Adamantyl)-4-cyclopropylmethoxyphenylethinyl]-benzoesäure
4-[2-Hexyloxy-5-(1-adamantyl)-4-hexyloxyphenylethinyl]-benzoesäure
4-[3-(1-Adamantyl)-4-heptyloxyphenylethinyl]-benzoesäure
6-[3-(1-Adamantyl)-4-methoxyethoxymethoxyphenylethinyl]-nicotinsäure
4-[2-Methoxy-5-(1-adamantyl)-4-methoxyphenylethinyl]-benzoesäure
4-[2-Methoxyethoxymethoxy-5-(1-adamantyl)-4-methoxyethoxymethoxyphenylethinyl)-benzoesäure
4-[2-Methoxy-5-(1-adamantyl)4-methoxyethoxymethoxyphenylethinyl]-benzoesäure
4-[3-1-Adamantyl)-4-benzyloxyphenylethinyl]-benzoesäure
4-[3-(1-Adamantyl)-4-methoxyethoxymethoxyphenylehtinyl]-benzaldehyd
4-[-3-(1-Adamantyl)-4-methoxyethoxymethoxyphenylethinyl]-benzolmethanol
4-[3-(1-Adamantyl)-4-methoxyethoxymethoxyphenylethinyl]-benzylacetat
4-[3-(1-Adamantyl)-4-methoxyethoxymethoxyphenylethinyl]-phenylacetat
4-[3-(1-Adamantyl)-4-methoxyethoxymethoxyphenylethinyl]-phenol
4-[3-(1-Adamantyl)-4-methoxyethoxymethoxyphenylethinyl]-phenoxyethylmorpholin
4-[3-(1-Adamantyl)-4-methoxyethoxymethoxyphenylethinyl]-benzamid
N-Ethyl-4-[3-(1-adamantyl)4-methoxyethoxymethoxyphenylethinyl]-benzamid
4-[3-(1-Adamantyl)-4-methoxyethoxymethoxyphenylethinyl]-benzoesäuremorpholid
2-Hydroxy-4-[3-(1-adamantyl)-4-methoxyethoxymethoxyphenylethinyl]-benzoesäure
4-[3-(1-Adamantyl)-4-(6-hydroxyhexyloxy)-phenylethinyl]-benzoesäure
4-[3-(1-Adamantyl)-4-(6-methoxyhexyloxy)-phenylethinyl]-benzoesäure
4-[[3-(1-Adamantyl)-4-[2-(4-morphalino)-ethoxy]-phenylethinyl]]-benzoesäure
4-[3-(1-Adamantyl)-4-(3-carbarmoylpropyloxy)-phenylethinyl]-benzoesäure
4-[3-(1-Adamantyl)-4-(5-carbarmoylpentyloxy)-phenylethinyl]-benzoesäure
4-[3-(1-Adamantyl)-4-(3-hydroxypropyloxy)-phenylethinyl]-benzoesäure
4-[3-(1-Adamantyl)-4-(3-hydroxy-2-methylpropyloxy)-phenylethinyl]-benzoesäure
4-[3-(1-Adamantyl)-4-(2,2-dimethyl-1,3-dioxolan-4-methoxy)-phenylethinyl]-benzoesäure
4-[3-(1-Adamantyl)-4-(2,3-dihydroxypropyloxy)-phenylethinyl]-benzoesäure
4-[3-(1-Adamantyl)-4-methoxyethoxyethylphenylethinyl]-benzoesäure
4-[3-(1-Adamantyl)-4-methoxymethoxyproylphenylethinyl]-benzoesäure
4-[3-(1-Adamantyl)-4-methoxyethoxypropylphenylethinyl]-benzoesäure
4-[3-(1-Adamantyl)-4-acetoxybutoxyphenylethinyl]-benzoesäure
4-[3-(1-Adamantyl)4-acetoxypropyloxyphenylethinyl]-benzoesäure.

17. Verbindungen nach Anspruch 1, dadurch gekennzeichnet, daß sie mindestens eine und vorzugsweise alle folgenden Eigenschaften aufweisen: R₁ ist eine Gruppe -(CH₂)ₚ-CO-R₈; R₃ ist eine Gruppe -O-CH₂-O-CH₂-CH₂-O-CH₃ oder -(Y)ₙ-(CH₂)_{q}-R₁₄; Ar ist eine Gruppe, die unter den Gruppen der Formel (a) und (e) ausgewählt ist; und R₂ ist eine geradkettige oder verzweigte Alkylgruppe mit 1 bis 20 Kohlenstoffatomen oder eine Gruppe -O-R₁₃.

18. Verbindungen nach einem der vorhergehenden Ansprüche für eine Verwendung als Arzneimittel.

19. Verbindungen nach Anspruch 18 für eine Verwendung als Arzneimittel, das zur Behandlung von dermatologischen Erkrankungen, rheumatischen Erkrankungen, Erkrankungen der Atemwege, kardiovaskulären Erkrankungen und ophthalmologischen Erkrankungen bestimmt ist, sowie zur Behandlung oder zur Vorbeugung von Osteoporose.

20. Verwendung mindestens einer der Verbindungen nach den Ansprüchen 1 bis 17 zur Herstellung eines Arzneimittels, das zur Behandlung von dermatologischen Erkrankungen, rheumatischen Erkrankungen, Erkrankungen der Atemwege, kardiovaskulären Erkrankungen und ophthalmologischen Erkrankungen sowie zur Behandlung oder zur Vorbeugung von Osteoporose bestimmt ist.

21. Pharmazeutische Zusammensetzung, dadurch gekennzeichnet, daß sie in einem pharmazeutisch akzeptablen Träger mindestens eine der Verbindungen nach einem der Ansprüche 1 bis 17 enthält.

22. Zusammensetzung nach Anspruch 21, dadurch gekennzeichnet, daß die Konzentration der Verbindung(en) nach einem der Ansprüche 1 bis 17 im Bereich von 0,001 bis 5 Gew.-%, bezogen auf die gesamte Zusammensetzung, liegt.

23. Kosmetische Zusammensetzung, dadurch gekennzeichnet, daß sie in einem kosmetisch akzeptablen Träger mindestens eine Verbindung nach einem der Ansprüche 1 bis 17 enthält.

24. Zusammensetzung nach Anspruch 23, dadurch gekennzeichnet, daß die Konzentration der Verbindung(en) nach einem der Ansprüche 1 bis 17 im Bereich von 0,001 bis 3 Gew.-%, bezogen auf die gesamte Zusammensetzung, liegt.

25. Verwendung einer kosmetischen Zusammensetzung nach einem der Ansprüche 23 oder 24 zur Körper- oder Haarhygiene.
